(19) Europäisches Patentamt
European Patent Office
Office européen des brevets

(11) EP 0 992 493 A1

(12) EUROPEAN PATENT APPLICATION

(43) Date of publication:
**12.04.2000 Bulletin 2000/15**

(21) Application number: **99307878.1**

(22) Date of filing: **06.10.1999**

(51) Int Cl.[7]: **C07D 211/28**, C07D 403/14, C07D 405/14, A61K 31/44, C07D 405/06, C07D 309/04

(84) Designated Contracting States:
**AT BE CH CY DE DK ES FI FR GB GR IE IT LI LU MC NL PT SE**
Designated Extension States:
**AL LT LV MK RO SI**

(30) Priority: **08.10.1998 GB 9821898**

(71) Applicants:
- **PFIZER INC.**
**New York, N.Y. 10017 (US)**
Designated Contracting States:
**BE CH DE DK ES FI FR GR IE IT LI LU MC NL PT AT**
- **Pfizer Limited**
**Sandwich, Kent CT13 9NH (GB)**
Designated Contracting States:
**GB**

(72) Inventor: **Fox, David Nathan Abraham**
**Sandwich, Kent CT13 9NJ (GB)**

(74) Representative:
**Simpson, Alison Elizabeth Fraser et al**
**Urquhart-Dykes & Lord,**
**91 Wimpole Street**
**London W1M 8AH (GB)**

(54) **Substituted benzoylpiperidine derivatives and their use as Neurokinin antagonists**

(57) The present invention provides a compound of the formula:-

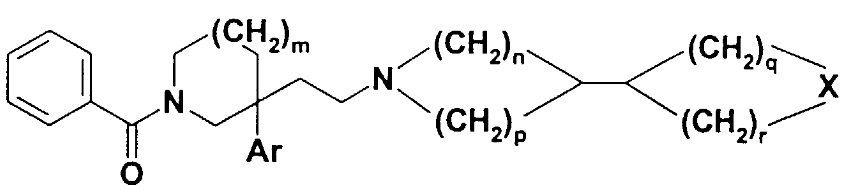

**(I)**

or a pharmaceutically acceptable acid addition salt thereof,
wherein

Ar is phenyl substituted by 1 or 2 substituent(s) each independently selected from fluoro and chloro;
X is $NSO_2(C_1\text{-}C_4$ alkyl), $NSO_2$(halo($C_1\text{-}C_4$ alkyl)) or O;
m is 0 or 1 ;
n is 1 or 2;
p is 1 or 2;
q is 1 or 2; and

r is 1 or 2, together with processes for the preparation of, intermediates used in the preparation of, compositions

EP 0 992 493 A1

containing and uses of, such derivatives. The compounds have tachykinin receptor antagonist activity.

## Description

This invention relates to heterocycles. More particularly, this invention relates to 1-benzoylpiperidine and 1-benzoylpyrrolidine derivatives and to processes for the preparation of, intermediates used in the preparation of, compositions containing and uses of, such derivatives.

The present compounds are antagonists of tachykinins, including neurokinin A (NKA), neurokinin B (NKB) and Substance P, acting at the human neurokinin-1 ($NK_1$), neurokinin-2 ($NK_2$) or neurokinin-3 ($NK_3$) receptor, or any combination thereof. The derivatives are therefore useful for treating an inflammatory disease such as arthritis, psoriasis, asthma or inflammatory bowel disease, a central nervous system (CNS) disorder such as anxiety, depression, dementia or psychosis, a gastro-intestinal (GI) disorder such as functional bowel disease, irritable bowel syndrome, gastro-oesophageal reflux, faecal incontinence, colitis or Crohn's disease, a disease caused by Helicobacter pylori or other urease-positive Gram negative bacteria, an urogenital tract disorder such as incontinence, impotence, hyperreflexia or cystitis, a pulmonary disorder such as chronic obstructive airways disease, an allergy such as eczema, contact dermatitis or rhinitis, a hypersensitivity disorder such as to poison ivy, a vasospastic disease such as angina or Reynaud's disease, a fibrosing or collagen disease such as scleroderma or eosinophillic fascioliasis, reflux sympathetic dystrophy such as shoulder/hand syndrome, an addiction disorder such as alcoholism, a stress-related somatic disorder, a peripheral neuropathy such as diabetic neuropathy, neuralgia, causalgia, painful neuropathy, a burn, herpetic neuralgia or post-herpetic neuralgia, a neuropathological disorder such as Alzheimer's disease or multiple sclerosis, a disorder related to immune enhancement or suppression such as systemic lupus erythematosis, a rheumatic disease such as fibrositis, emesis, cough, acute or chronic pain, migraine, or an opthalmic disease such as proliferative retinopathy.

WO-A-97/25322 discloses azetidinylalkyl-piperidine, -homopiperidine and -pyrrolidine derivatives having tachykinin receptor antagonist activity.

EP-A-0714891 discloses heterocyclic tachykinin receptor antagonists.

WO-A-97/10211 discloses compounds having selective human $NK_3$ receptor antagonist activity.

With respect to this prior art, the present compounds have been surprisingly found to be more potent $NK_2$ receptor antagonists and/or to have increased metabolic stability and/or to have improved selectivity as antagonists for the $NK_2$ receptor as opposed to the $NK_3$ receptor.

As stated above, the present compounds are particularly potent and selective antagonists of tachykinins, including NKA, NKB and Substance P, acting at the human $NK_2$ receptor. They are therefore particularly useful for treating an inflammatory disease such as arthritis, psoriasis, asthma or inflammatory bowel disease, a central nervous system (CNS) disorder such as anxiety, depression, dementia or psychosis, a gastro-intestinal (GI) disorder such as functional bowel disease, irritable bowel syndrome, gastro-oesophageal reflux, faecal incontinence, colitis or Crohn's disease, an urogenital tract disorder such as incontinence, hyperreflexia or cystitis, a pulmonary disorder such as chronic obstructive airways disease, an allergy such as eczema, contact dermatitis or rhinitis, a hypersensitivity disorder such as to poison ivy, a peripheral neuropathy such as diabetic neuropathy, neuralgia, causalgia, painful neuropathy, a burn, herpetic neuralgia or post-herpetic neuralgia, cough or acute or chronic pain.

The present invention provides a compound of the formula:-

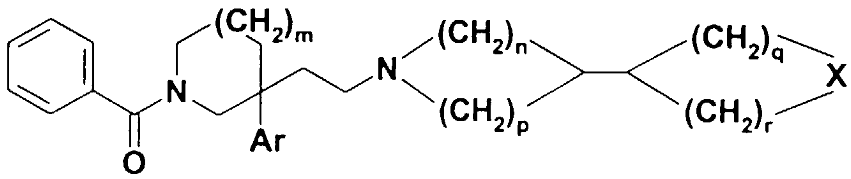

(I)

or a pharmaceutically acceptable acid addition salt thereof,
wherein

- Ar is phenyl substituted by 1 or 2 substituent(s) each independently selected from fluoro and chloro;
- X is $NSO_2(C_1\text{-}C_4\ alkyl)$, $NSO_2(halo(C_1\text{-}C_4\ alkyl))$ or O;
- m is 0 or 1 ;

n is 1 or 2;
p is 1 or 2;
q is 1 or 2; and
r is 1 or 2.

In the above definition of a compound of the formula (I), an alkyl or haloalkyl group containing 3 or 4 carbon atoms may be straight- or branched-chain and halo means fluoro, chloro, bromo or iodo. An example of a halo($C_1$-$C_4$alkyl) group is trifluoromethyl.

Preferably, Ar is 4-chlorophenyl, 3,4-dichlorophenyl, 3,4-difluorophenyl or 4-chloro-3-fluorophenyl.

Preferably, X is $NSO_2(C_1$-$C_4$ alkyl) or O.

Preferably, X is $NSO_2CH_3$ or O.

Preferably, n is 1 and p is 1.

Preferably, n is 2 and p is 2.

Preferably, q is 1 and r is 1.

Preferably, q is 2 and r is 2.

Preferably, the group

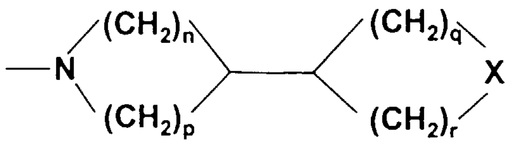

for a compound of the formula (I) is

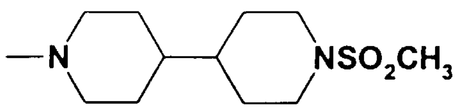

,

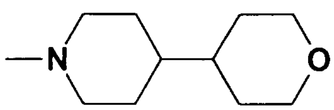

,

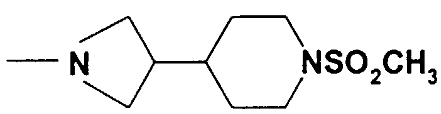

or

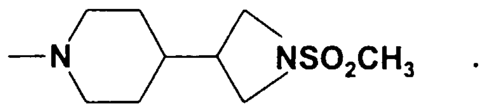

Suitable acid addition salts are formed from acids which form non-toxic salts and examples include the hydrochloride, hydrobromide, hydroiodide, sulphate, hydrogen sulphate, nitrate, phosphate, hydrogen phosphate, acetate, maleate, fumarate, lactate, tartrate, citrate, gluconate, succinate, benzoate, methanesulphonate, benzenesulphonate, p-toluenesulphonate, 5-sulphosalicylate and 10-camphorsulphonate salts.

For a review on suitable acid addition salts that may be used see Berge et al, J. Pharm. Sci., 66, 1-19 (1977).

Also included within the present scope of the compounds of the formula (I) are solvates, polymorphs and radiolabelled derivatives thereof.

The pharmaceutically acceptable solvates of the compounds of the formula (I) include the hydrates thereof.

A compound of the formula (I) contains at least one asymmetric carbon atom and therefore exists in two or more stereoisomeric forms. The present invention includes the individual stereoisomers of the compounds of the formula (I) and mixtures thereof.

Separation of diastereoisomers may be achieved by conventional techniques, e.g. by fractional crystallisation, chromatography or H.P.L.C. of a stereoisomeric mixture of a compound of the formula (I) or a suitable salt or derivative thereof. An individual enantiomer of a compound of the formula (I) may also be prepared from a corresponding optically pure intermediate or by resolution, such as by H.P.L.C. of the corresponding racemate using a suitable chiral support or by fractional crystallisation of the diastereoisomeric salts formed by reaction of the corresponding racemate with a suitable optically active acid.

The preferred compounds of the formula (I) have the (S)-stereochemistry as shown below:

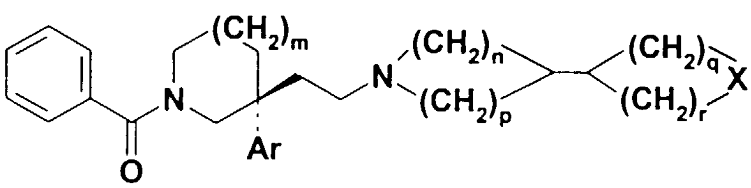

(IA)

wherein Ar, X, m, n, p, q and r are as previously defined for a compound of the formula (I).

Preferred compounds of the formula (I) are those of the Examples hereafter, including pharmaceutically acceptable acid addition salts thereof.

The compounds of the formula (I) provided by the invention can be prepared by the following methods wherein Ar, X, m, n, p, q and r are as previously defined for a compound of the formula (I) unless otherwise stated.

1) The compounds of the formula (I) can be prepared by reductive amination using as starting materials a compound of the formula:-

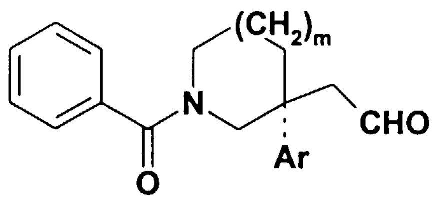

(II)

and a compound of the formula:-

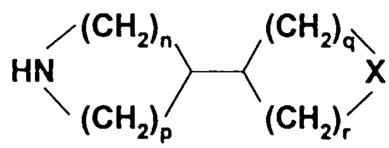

(III)

, or an acid addition salt thereof. The reaction may be carried out in the presence of a suitable acid, e.g. acetic acid.

The reaction proceeds via the initial formation of an intermediate iminium salt of the formula:-

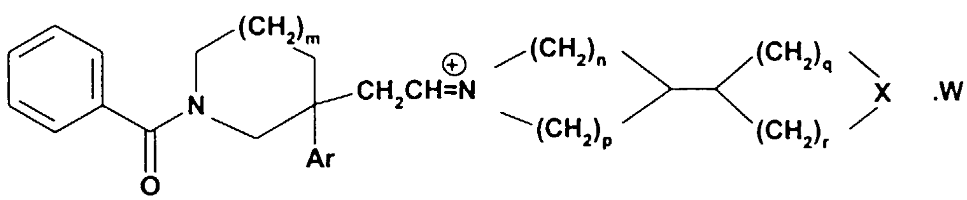

(IV)

where W is an anion (e.g. acetate) derived from a suitable acid (e.g. acetic acid), if present, which may be stable and isolatable. The reaction is preferably carried out without isolation of the intermediate of the formula (IV) in which case it is reduced in situ to provide a compound of formula (I).

In a typical procedure, an aldehyde of the formula (II) is mixed with a compound of the formula (III) in a suitable solvent, e.g. tetrahydrofuran or dichloromethane, and the mixture is then treated with a suitable reducing agent, e.g. sodium triacetoxyborohydride or sodium cyanoborohydride, optionally in the presence of a suitable acid, e.g. acetic acid, to give the required product. If an acid addition salt of a compound of the formula (III) is used as a starting material, a suitable acid acceptor, e.g. triethylamine, may be added prior to the addition of the reducing agent.

The reaction is typically carried out at room temperature.

The compounds of the formulae (II) and (III) can generally be prepared by conventional procedures as illustrated in the Preparations section hereafter. Alternatively, the compounds of the formula (III) may be prepared by modification of synthetic procedures described in Synlett, 1998, 379-380.

2) The compounds of the formula (I) can be prepared by N-benzoylation of a compound of the formula:

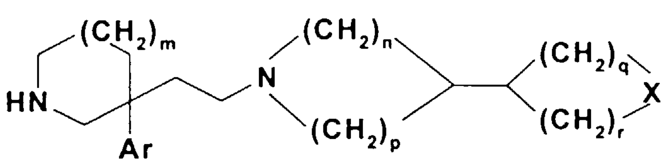

(V)

with a compound of the formula:

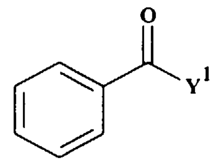

(VI)

wherein $Y^1$ is a suitable leaving group, e.g. halo, preferably chloro or bromo, hydroxy or $C_1$-$C_4$ alkoxy.

In a typical procedure, where $Y^1$ is halo, the reaction can be carried out by reacting the compounds of the formulae (V) and (VI) together in the presence of a suitable acid acceptor e.g. triethylamine, and in a suitable solvent, e.g. dichloromethane, at room temperature. If a compound of the formula (VI) where $Y^1$ is chloro is used, potassium iodide may also be added to increase the rate of reaction.

Where $Y^1$ is hydroxy, conventional peptide coupling techniques may be used.

The compounds of the formula (V) can be prepared by conventional procedures.

The compounds of the formula (VI) are either known compounds or can be prepared by conventional methods.

3) The compounds of the formula (I) can be prepared by reaction of a compound of the formula:

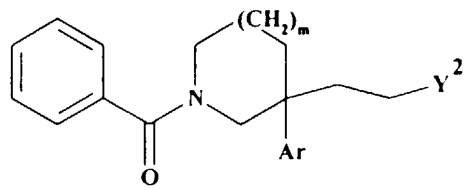

(VII)

wherein $Y^2$ is a suitable leaving group, e.g. chloro, bromo, iodo, methanesulphonyloxy, trifluoromethanesulphonyloxy or p-toluenesulphonyloxy, with a compound of the formula (III), or an acid addition salt thereof. Where an acid addition salt is used, the compound of the formula (III) may be generated in situ in the presence of a suitable acid acceptor, e. g. triethylamine.

In a typical procedure, a compound of the formula (VII) is reacted with a compound of the formula (III), or an acid addition salt thereof, in the presence of a suitable acid acceptor, e.g. triethylamine or potassium carbonate, and in a suitable solvent, e.g. acetonitrile.

The starting materials of the formula (VII) can be prepared by conventional methods such as by those described in WO-A-98/07722 and WO-A-97/25322 or by analogous preparations thereto.

All of the above reactions and the preparations of novel starting materials used in the preceding methods are conventional and appropriate reagents and reaction conditions for their performance or preparation as well as procedures for isolating the desired products will be well known to those skilled in the art with reference to literature precedents and the Examples and Preparations hereto.

A pharmaceutically acceptable acid addition salt of a compound of the formula (I) may be readily prepared by mixing together solutions of a compound of the formula (I) and the desired acid. The salt may precipitate from solution and be collected by filtration or may be recovered by evaporation of the solvent.

The affinity of the compounds of formula (I) and their salts for the human $NK_1$ receptor can be tested in vitro by testing their ability to inhibit [$^3$H]-Substance P binding to membranes prepared from the human IM9 cell line expressing the human $NK_1$ receptor using a modification of the method described in McLean, S. et al, J. Pharm. Exp. Ther., 267, 472-9 (1993) in which whole cells were used.

The affinity of the compounds of formula (I) and their salts for the human $NK_2$ receptor can be tested in vitro by testing their ability to compete with [$^3$H]-NKA (neurokinin A) for binding to membranes prepared from Chinese hamster ovary cells expressing the cloned human $NK_2$ receptor. In this method, washed Chinese hamster ovary cell membranes are prepared as described for the previous method where IM9 cells are used instead. The membranes are incubated (90 min, 25°C) with [$^3$H]-NKA and with a range of concentrations of the test compound. Non-specific binding was determined in the presence of 10μM NKA.

The $NK_2$ receptor antagonist activity of the compounds of the formula (I) can be tested, in vitro, by testing their ability to antagonise the contractile effects of the selective $NK_2$ receptor agonist [βAla$^8$]$NKA_{(4-10)}$ in the rabbit

pulmonary artery, using the method of Patacchini and Maggi, Eur. J. Pharmacol., 236, 31-37 (1993).

The compounds of the formula (I) and their salts can be tested for $NK_2$ receptor antagonist activity, in vivo, by testing their ability to inhibit bronchoconstriction induced by [βAla$^8$]$NKA_{(4-10)}$ in the anaesthetised guinea pig, using the method described by Murai et al, J. Pharm. Exp. Ther., 262, 403-408 (1992) or Metcalfe et al, Br. J. Pharmacol., 112, 563P (1994).

The compounds of the formula (I) and their salts can be tested for $NK_3$ receptor affinity, in vitro, by testing their ability to inhibit [$^3$H]-senktide binding to membranes from guinea pig cortex using the method of Guard, et al, Br. J.Pharmacol., 99, 767-773 (1990).

The in vitro metabolic stability of the compounds of the formula (I) and their salts can be tested by incubating samples for 1 hour at 37°C in the presence of a "moderate activity" (re. concentrations of cytochrome p450 enzymes present) human liver microsomal preparation (prepared from human liver homogenates). The samples were analysed by HPLC at regular intervals during this one hour period and the half-life (t½) determined (in minutes).

For human use, the compounds of the formula (I) and their salts can be administered alone, but will generally be administered in admixture with a pharmaceutically acceptable diluent or carrier selected with regard to the intended route of administration and standard pharmaceutical practice. For example, they can be administered orally, including sublingually and buccally, in the form of tablets containing such excipients as starch or lactose, or in capsules or ovules either alone or in admixture with excipients, or in the form of elixirs, solutions or suspensions containing flavouring or colouring agents. They can be injected parenterally, for example, intravenously, intramuscularly or subcutaneously. For parenteral administration, they are best used in the form of a sterile aqueous solution which may contain other substances, for example, enough salts or glucose to make the solution isotonic with blood.

For oral and parenteral administration to human patients, the daily dosage level of the compounds of the formula (I) and their salts will be from 0.001 to 20, preferably from 0.01 to 20, and most preferably from 0.1 to 10, mg/kg (in single or divided doses). Thus tablets or capsules of the compounds will contain from 0.1 to 500, preferably from 10 to 200, mg of active compound for administration singly or two or more at a time, as appropriate. The physician in any event will determine the actual dosage which will be most suitable for an individual patient and it will vary with the age, weight and response of the particular patient. The above dosages are exemplary of the average case; there can, of course, be individual instances where higher or lower dosage ranges are merited, and such are within the scope of this invention.

Alternatively, the compounds of the formula (I) can be administered intranasally, by inhalation or in the form of a suppository or pessary, or they may be applied topically in the form of a lotion, solution, cream, ointment or dusting powder. An alternative means of transdermal administration is by use of a skin patch. For example, they can be incorporated into a cream comprising an aqueous emulsion of polyethylene glycols or liquid paraffin, or they can be incorporated, at a concentration of between 1 and 10% by weight, into an ointment consisting of a white wax or white soft paraffin base together with such stabilisers and preservatives as may be required. The compounds of the formula (I) may also be administered in the form of an aerosol spray presentation or by using a dry powder inhaler formulation.

It is to be appreciated that reference to treatment includes curative, palliative and prophylactic treatment.

Thus the invention provides:-

(i) a compound of the formula (I) or a pharmaceutically acceptable acid addition salt thereof;
(ii) processes for the preparation of a compound of the formula (I) or a pharmaceutically acceptable acid addition salt thereof;
(iii) a pharmaceutical composition comprising a compound of the formula (I), or a pharmaceutically acceptable acid addition salt thereof, together with a pharmaceutically acceptable diluent or carrier;
(iv) a compound of the formula (I), or a pharmaceutically acceptable acid addition salt or composition thereof, for use as a medicament;
(v) the use of a compound of the formula (I), or of a pharmaceutically acceptable acid addition salt or composition thereof, for the manufacture of a medicament for the treatment of a disease by producing an antagonist effect on a tachykinin acting at the human $NK_1$, $NK_2$ or $NK_3$ receptor, or any combination thereof;
(vi) use as in (v) where the disease is an inflammatory disease such as arthritis, psoriasis, asthma or inflammatory bowel disease, a central nervous system (CNS) disorder such as anxiety, depression, dementia or psychosis, a gastro-intestinal (GI) disorder such as functional bowel disease, irritable bowel syndrome, gastro-oesophageal reflux, faecal incontinence, colitis or Crohn's disease, an urogenital tract disorder such as incontinence, hyperreflexia or cystitis, a pulmonary disorder such as chronic obstructive airways disease, an allergy such as eczema, contact dermatitis or rhinitis, a hypersensitivity disorder such as to poison ivy, a peripheral neuropathy such as diabetic neuropathy, neuralgia, causalgia, painful neuropathy, a burn, herpetic neuralgia or post-herpetic neuralgia, cough or acute or chronic pain;
(vii) a method of treatment of a human to treat a disease by producing an antagonist effect on a tachykinin acting at the human $NK_1$, $NK_2$ or $NK_3$ receptor, or any combination thereof, which comprises treating said human with

an effective amount of a compound of the formula (I) or with a pharmaceutically acceptable acid addition salt or composition thereof;

(viii) a method as in (vii) where the disease is an inflammatory disease such as arthritis, psoriasis, asthma or inflammatory bowel disease, a central nervous system (CNS) disorder such as anxiety, depression, dementia or psychosis, a gastro-intestinal (GI) disorder such as functional bowel disease, irritable bowel syndrome, gastro-oesophageal reflux, faecal incontinence, colitis or Crohn's disease, an urogenital tract disorder such as incontinence, hyperreflexia or cystitis, a pulmonary disorder such as chronic obstructive airways disease, an allergy such as eczema, contact dermatitis or rhinitis, a hypersensitivity disorder such as to poison ivy, a peripheral neuropathy such as diabetic neuropathy, neuralgia, causalgia, painful neuropathy, a burn, herpetic neuralgia or post-herpetic neuralgia, cough or acute or chronic pain; and

(ix) a compound of the formula (III), or an acid addition salt thereof, (IV) or (V).

The following Examples illustrate the preparation of the compounds of the formula (I).

Example 1

3(S)-1-Benzoyl-3-(3,4-dichlorophenyl)-3-{2-[4-(1-methylsulphonylpiperidin-4-yl)piperidin-1-yl]ethyl}piperidine

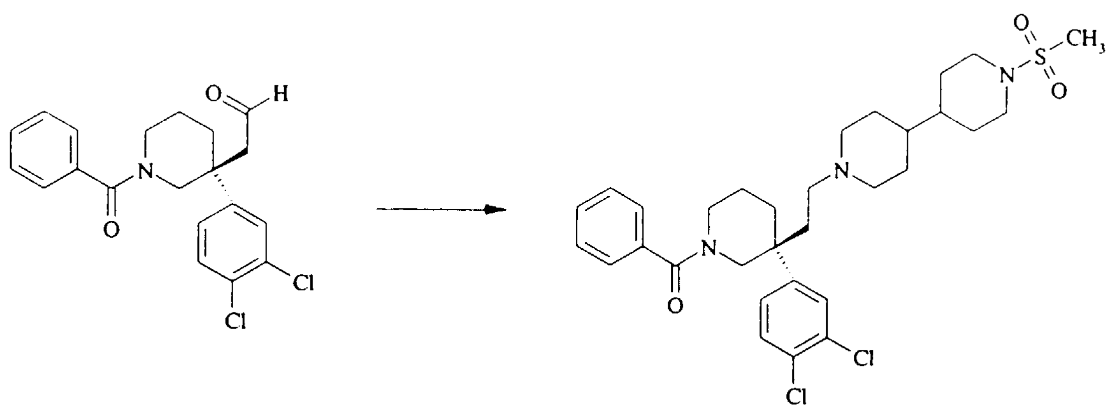

To a solution of 3(S)-1-benzoyl-3-(3,4-dichlorophenyl)-3-(formylmethyl)piperidine (Preparation 2) (300mg, 0.80mmol) and 1-methylsulfonyl-4-(piperidin-4-yl)piperidine (Preparation 28) (188mg, 0.75mmol) in dichloromethane (10ml) under nitrogen was added sodium triacetoxyborohydride (243mg, 1.15mmol) and the mixture was stirred for 2 hours. The solvent was removed under reduced pressure and the residue was partitioned between water and ethyl acetate. The organic layer was washed with brine, dried over $MgSO_4$, filtered and solvent removed under reduced pressure. The residue was taken up in diethyl ether and solvent removed under reduced pressure to afford the title compound as a white solid (456mg, 94%).

$^1$H-NMR ($CDCl_3$): $\delta$ = 7.40 (5H, bm), 7.30 (3H, bm), 3.75 (3H, bm), 3.50 (3H, bm), 3.25 (2H, bs), 2.7 (3H, s), 2.50 (2H, t), 2.60-2.10 (4H, bm), 2.10-1.60 (9H, bm), 1.25 (1H, bm) 1.20 (6H, m).

m/z: 606 ($MH^+$).

Examples 2-5

The compounds of the following tabulated Examples of the general formula :

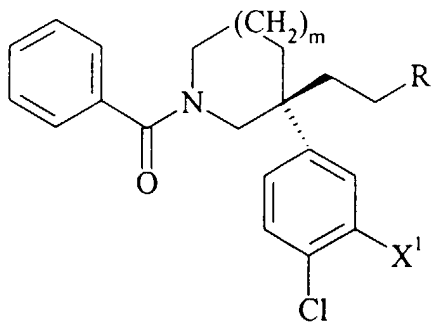

were prepared by a similar method to Example 1 using the appropriate aldehyde and amine.

| Ex. No. | Aldehyde Prep. No. | R | $X^1$ | m | Analytical Data |
|---|---|---|---|---|---|
| 2 | 2 | 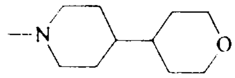 (see Preparation 29 for amine preparation) | Cl | 1 | $^1$H-NMR ($d_6$-DMSO): δ = 7.60 (2H, m), 7.40 (4H, m), 7.20 (2H, m), 3.80 (2H, m), 3.50 (1H, bm), 3.35 (2H, m), 3.20 (5H, m), 2.60 (2H, bm), 2.10 (3H, bm), 1.90-1.50 (5H, bm), 1.45 (2H, m), 1.40-1.0 (8H, m). m/z: 529 ($MH^+$). |
| 3 | 20 | 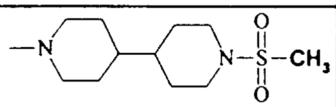 (see Preparation 28 for amine preparation) | H | 0 | $^1$H-NMR ($CD_3OD$): δ = 7.50 (5H, m), 7.40 (3H, m), 7.20 (1H, m), 4.10-3.80 (2H, m), 3.75 (3H, m), 3.60 (1H, m) 3.30 (2H,m), 2.80 (3H, m), 2.65 (4H, bm), 2.50-2.20 (4H, bm), 2.00 (2H, m), 1.80 (4H, bm), 1.30 (6H, bm). m/z: 558 ($MH^+$). |
| 4 | WO-A-97/25322 | 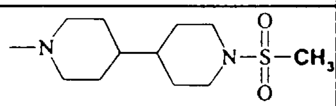 (see Preparation 28 for amine preparation) | Cl | 0 | $^1$H-NMR ($CD_3OD$): δ = 7.50 (5H, m), 7.40 (2H, m), 7.20 (1H, m), 4.00-3.80 (2H, m), 3.75 (3H, m), 3.60 (1H, m) 3.30 (2H, m), 2.80 (3H, m), 2.65 (2H, bm), 2.60-2.10 (7H, bm), 2.00 (1H, m), 1.80 (4H, bm), 1.30 (6H, bm). m/z: 594 ($MH^+$). |

| | | | | | |
|---|---|---|---|---|---|
| 5 | 20 | 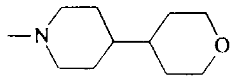 (see Preparation 29 for amine preparation) | H | 0 | $^1$H-NMR ($CDCl_3$): δ = 7.50 (2H, m), 7.40 (4H, m), 7.25 (2H, m), 7.10 (1H, m), 3.95 (3H, m), 3.90-3.60 (3H, m), 3.30 (4H, m), 2.70 (1H, m), 2.50-2.10 (7H, m), 2.00-1.70 (4H, bm), 1.60 (2H, m), 1.40-1.0 (4H, m). m/z: 481 ($MH^+$). |

Example 6

3(S)-1-Benzoyl-3-(3,4-dichlorophenyl)-3-{2-[3-(1-methylsulphonyl-4-piperidinyl)azetidin-1-yl]ethyl}piperidine hydrochloride

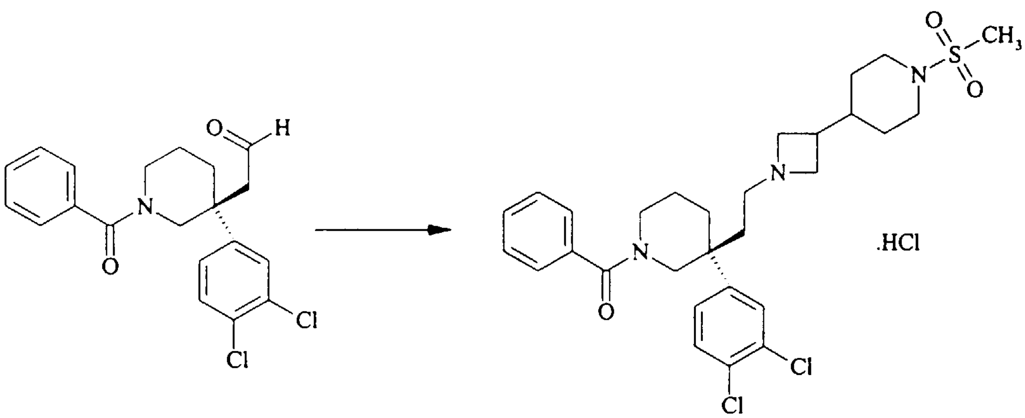

The title compound was prepared by a similar method to Example 1 from 3(S)-1-benzoyl-3-(3,4-dichlorophenyl)-3-(formylmethyl)piperidine (Preparation 2) and 4-(azetidin-3-yl)-1-(methylsulfonyl)piperidine (Preparation 25). The residue was purified by column chromatography on silica gel eluting with a solvent system of dichloromethane : methanol : 0.88 ammonia ( 93 : 7 : 1, by volume). The product was taken up in dichloromethane, a solution of hydrogen chloride (1N) in diethyl ether was added and the solvent was removed under reduced pressure to afford the title compound as a white solid.

$^1$H-NMR ($CDCl_3$): δ = 7.50 (5H, m), 7.40 (2H, m), 7.20 (1H, m), 4.40 (1H, bs), 4.00 (1H, s), 3.90-3.40 (5H, bm), 3.20 (2H, m), 2.75 (3H, m), 2.65 (2H, bm), 2.60-2.30 (4H, bm), 2.05 (4H, m), 1.85 (1H, bm), 1.65 (4H, bm) 1.50 (1H, bm), 1.15 (2H, bm).

m/z: 578 ($MH^+$).

Example 7

3(S)-1-Benzoyl-3-(3,4-difluorophenyl)-3-{2-[4-(1-methylsulphonylpiperidin-4-yl)piperidin-1-yl]ethyl}piperidine

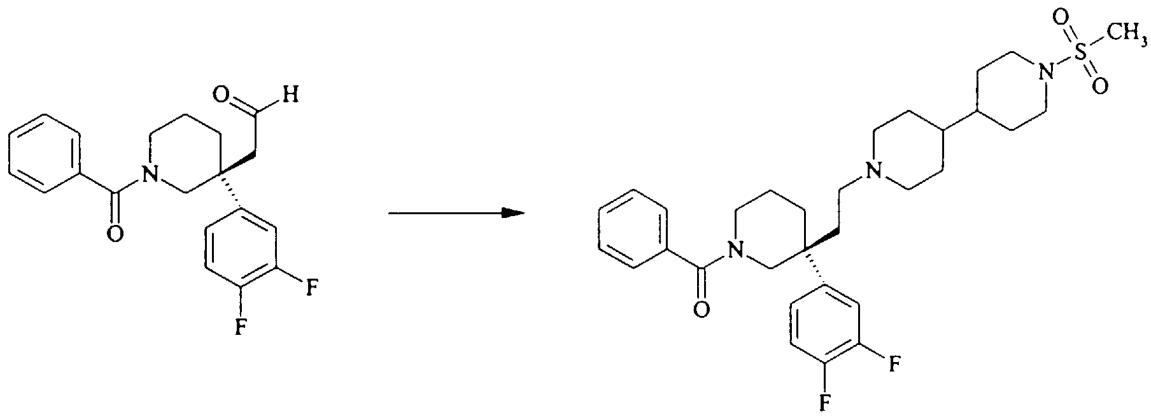

To a solution of 3(S)-1-benzoyl-3-(3,4-difluorophenyl)-3-(formylmethyl)piperidine (Preparation 15) (163mg, 0.48mmol) and 1-methylsulfonyl-4-(piperidin-4-yl)piperidine (Preparation 28) (117mg, 0.48mmol) in dichloromethane (5ml) under nitrogen was added sodium triacetoxyborohydride (151mg, 0.71mmol) and the mixture was stirred for 18 hours. The reaction mixture was quenched with aqueous hydrochloric acid (2N), the solvent was removed under reduced pressure and the residue was partitioned between aqueous sodium hydroxide solution (2N) and ethyl acetate. The organic layer was washed with brine, dried over $MgSO_4$, filtered and solvent removed under reduced pressure. The crude product was purified by column chromatography on silica gel eluting with a solvent gradient of dichloromethane gradually changing to dichloromethane : methanol : 0.88 ammonia (95 : 5 : 1, by volume) to afford the title compound as a white solid (140mg, 51%).
$^1$H-NMR ($CDCl_3$): $\delta$ = 7.40 (4H, m), 7.20 (2H, m), 7.10 (2H, m), 4.55 (1H, bs), 3.80 (2H, m), 3.50 (2H, m), 3.25 (1H, m), 3.00-2.65 (5H, m), 2.60 (2H, m), 2.20 (2H, m), 2.05-1.50 (12H, m) 1.50-1.95 (6H, m).
m/z: 574 ($MH^+$).

Example 8

3(S)-1-Benzoyl-3-(3,4-difluorophenyl)-3-{2-[4-(tetrahydropyran-4-yl)piperidin-1-yl]ethyl}piperidine

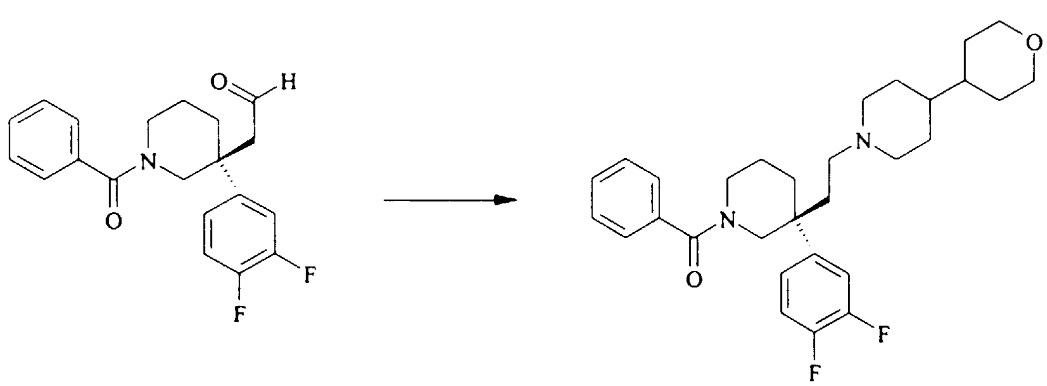

The title compound was prepared by a similar method to Example 7 from 3(S)-1-benzoyl-3-(3,4-difluorophenyl)-3-(formylmethyl)piperidine (Preparation 15) and 4-(tetrahydropyran-4-yl)piperidine (Preparation 29) to afford the title compound as a white solid (40%).
$^1$H-NMR ($CDCl_3$): δ = 7.40 (3H, bm), 7.20 (2H, bm), 7.10 (3H, bm), 3.95 (2H, dd), 3.50 (1H, bm), 3.30 (3H, bm), 2.95 (2H, bm), 2.40-1.75 (8H, bm), 1.75-1.50 (6H, bm), 1.40-1.10 (6H, bm) 1.05 (1H, bm), 0.9 (1H, bm).
m/z: 497 ($MH^+$).

Example 9

3(S)-1-Benzoyl-3-(4-chlorophenyl)-3-{2-[4-(tetrahydropyran-4-yl)piperidin-1-yl]ethyl}piperidine

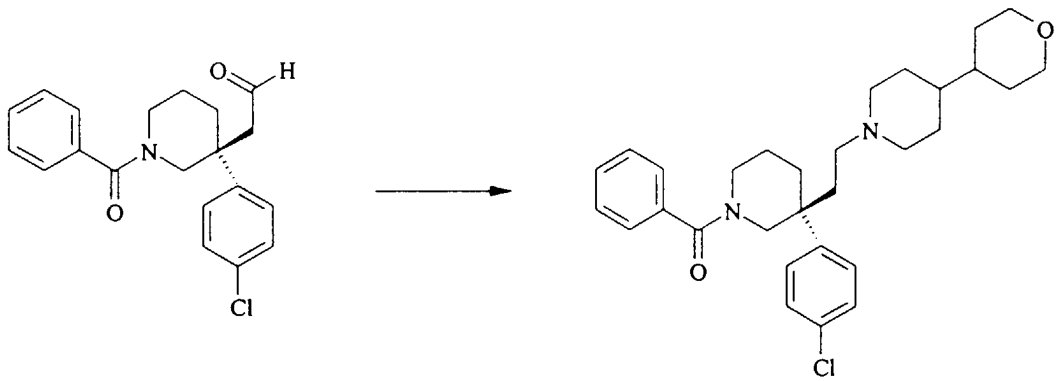

The title compound was prepared by a similar method to Example 7 from 3(S)-1-benzoyl-3-(4-chlorophenyl)-3-(formylmethyl)piperidine (Preparation 9) and 4-(tetrahydropyran-4-yl)piperidine (Preparation 29) to afford the title compound as a white solid (98%).
$^1$H-NMR ($CDCl_3$): δ = 7.50-7.0 (9H, bm), 3.95 (2H, dd), 3.45 (1H, bm), 3.30 (3H, bm), 2.80 (2H, bm), 2.20 (2H, bm), 2.00-1.70 (6H, bm), 1.70-1.50 (6H, bm), 1.40-1.10 (6H, bm) 1.00 (2H, bm).
m/z: 497 ($MH^+$).

Example 10

3(S)-1-Benzoyl-3-(3,4-dichlorophenyl)-3-{2-[4-(1-methylsulfonylazetidin-3-yl)piperidin-1-yl]ethyl}piperidine

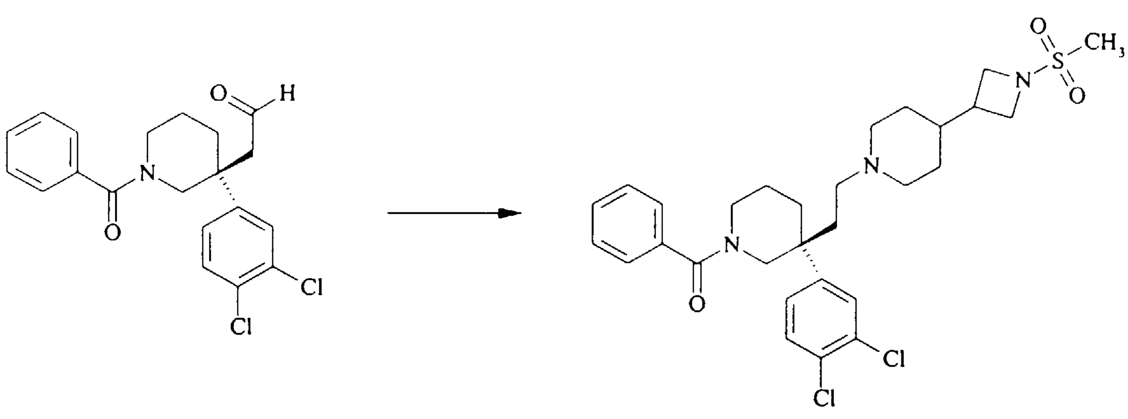

The title compound was prepared by a similar method to Example 7 from 3(S)-1-benzoyl-3-(3,4-dichlorophenyl)-3-(formylmethyl)piperidine (Preparation 2) and 4-[1-methylsulfonylazetidin-3-yl]piperidine (Preparation 36) to afford the title compound as a white solid (67%).
$^1$H-NMR ($CDCl_3$): δ = 7.60-7.20 (8H, m), 4.60 (1H, bs), 3.90 (2H, t), 3.65 (2H, t), 3.60-3.10 (4H, m), 3.00-2.70 (4H, m),

2.35 (1H, bm), 2.15 (2H, m), 2.10-1.70 (6H, m), 1.60 (3H, m), 1.40 (2H, m) 1.35-1.00 (2H, m).
m/z: 578, 580 ($MH^+$).

The following Preparations illustrate the preparation of certain intermediates used in the preceding Examples.

Preparation 1

3(S)-1-Benzoyl-3-(3,4-dichlorophenyl)-3-(1,3-dioxolan-2-ylmethyl)piperidine

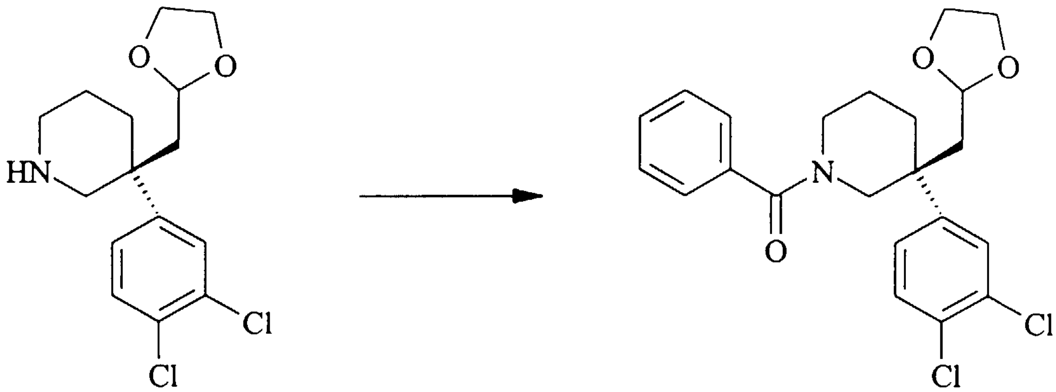

Benzoyl chloride (1.83ml, 15.8mmol) was added to a solution of 3(S)-3-(3,4-dichlorophenyl)-3-(1,3-dioxolan-2-ylmethyl)piperidine (WO-A-97/25322) (5.0g, 15.8mmol) and triethylamine (2.23ml, 16.0mmol) in tetrahydrofuran (150ml). The reaction mixture was stirred under nitrogen at room temperature for 24 hours. The solvent was removed under reduced pressure and the residue partitioned between ethyl acetate and water. The organic layer was separated and washed with saturated aqueous sodium carbonate solution, brine, dried over $MgSO_4$, filtered and solvent removed under reduced pressure. The residue was purified by column chromatography on silica gel eluting with a gradient system of hexane gradually changing to hexane : ethyl acetate (1 : 3, by volume) to afford the title compound as a white solid (6.1g, 92%).
$^1$H-NMR ($CDCl_3$): $\delta$ = 7.40 (5H, bm), 7.30 (3H, bm), 4.80 (1H, bm), 4.40 (1H, bm), 3.90 (2H, m), 3.70 (2H, m), 3.50 (1H, bm), 3.40 (1H, bm), 3.20 (1H, bm), 2.25 (1H, m), 2.05 (1H, m), 1.95 (2H, bm), 1.50 (2H, bm).
m/z : 420 ($MH^+$)

Preparation 2

3(S)-1-Benzoyl-3-(3,4-dichlorophenyl)-3-(formylmethyl)piperidine

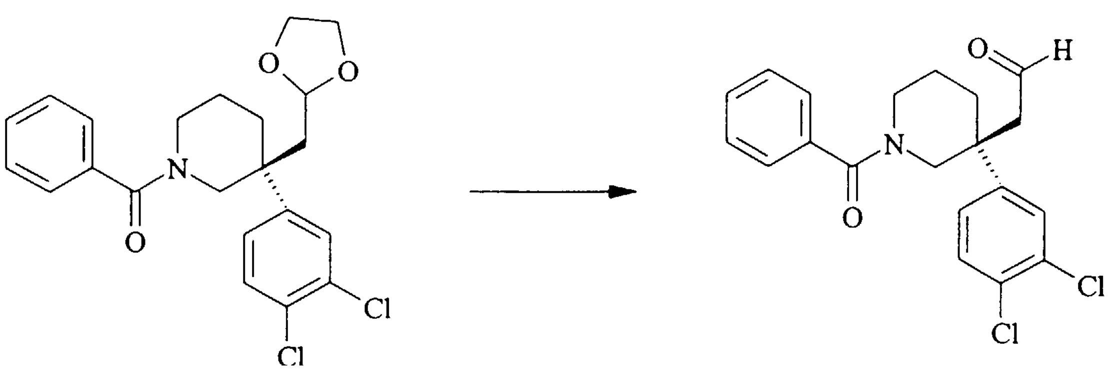

Amberlyst (trade mark) 15 ion exchange resin (2g) was added to a solution of 3(S)-1-benzoyl-3-(3,4-dichlorophenyl)-3-(1,3-dioxolan-2-ylmethyl)piperidine (Preparation 1) (5.95g, 14.16mmol) in methanol (60ml) and stirred at room temperature for 24 hours. The reaction mixture was filtered through Arbocel (trade mark) filter aid and the solvent removed under reduced pressure. The residue was added to a solution of aqueous hydrochloric acid (1N, 15ml) in tetrahydrofuran (15ml) and stirred at room temperature for 3 days. The tetrahydrofuran was removed under reduced pressure and the aqueous layer was extracted with ethyl acetate (x2). The combined organic layers were washed with brine, dried over $MgSO_4$, filtered and solvent removed under reduced pressure. The residue was purified by column chromatography on silica gel eluting with a gradient system of ethyl acetate : hexane (1 : 1, by volume) gradually changing to ethyl acetate to afford the title compound as a colourless oil (5.02g, 94%).

$^1$H-NMR ($CDCl_3$): δ = 9.50 (1H, bs), 7.40 (5H, m), 7.30 (3H, m), 4.10 (1H, bm), 3.90 (1H, bm), 3.40 (2H, m), 2.75 (2H, m), 2.20 (1H, m), 2.10 (1H, m), 1.60 (2H, bm).

m/z: 377 ($MH^+$).

Preparation 3

2-(4-Chlorophenyl)-3-(1,3-dioxolan-2-yl)propanenitrile

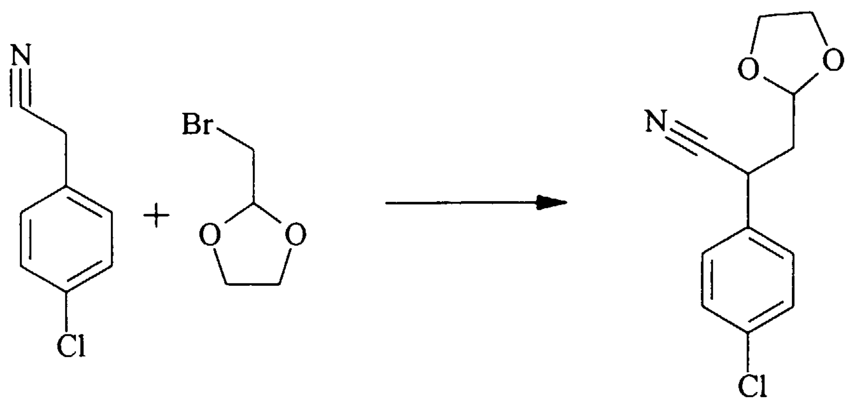

A solution of 4-chlorobenzyl cyanide (10g, 66.0mmol) in tetrahydrofuran (45ml) was added dropwise to a suspension of sodium hydride (60 % w/w dispersion in mineral oil, 2.91g, 72.75mmol) in tetrahydrofuran (25ml) at 0°C. The reaction mixture was allowed to warm to room temperature and stirred for 18 hours. 2-Bromomethyl-1,3-dioxolane (12.1g, 72.75mmol) was added to the reaction mixture followed by tetra-n-butylammonium iodide (1g, 2.7mmol) and then the mixture heated under reflux for 4 hours. The reaction mixture was cooled and partitioned between ethyl acetate and water, the layers were separated and aqueous layer re-extracted with ethyl acetate. The combined organic layers were washed with brine, dried over $MgSO_4$, filtered and solvent was removed under reduced pressure. The residue was purified by column chromatography on silica gel eluting with a solvent system of hexane : ethyl acetate (11: 2, by volume) to afford the title compound as an orange oil (11.6g, 74%).

$^1$H-NMR ($CDCl_3$): δ = 7.30 (4H, m), 4.95 (1H, m), 4.00 (3H, m), 3.95 (2H, m), 2.30 (1H, m), 2.10 (1H, m).

Preparation 4

Ethyl 4-(4-chlorophenyl)-4-cyano-5-(1,3-dioxolan-2-yl)pentanoate

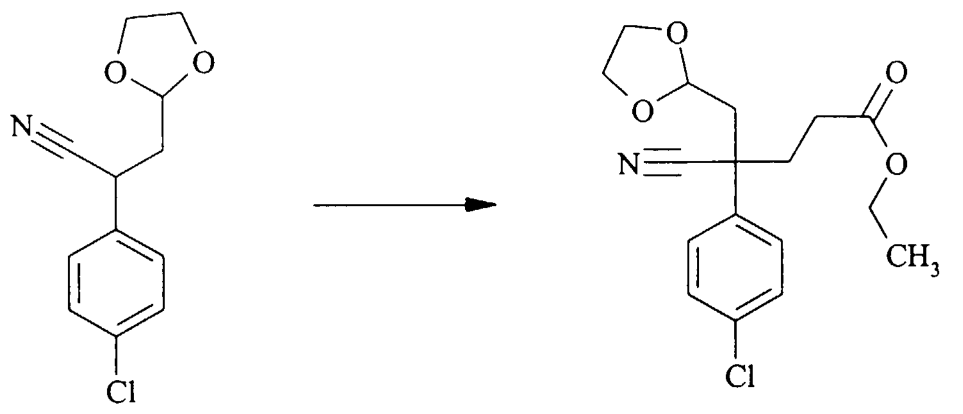

A solution of lithium diisopropylamide in tetrahydrofuran (1.5M, 10.44ml, 15.65mmol) was added to a solution of 2-(4-chlorophenyl)-3-(1,3-dioxolan-2-yl)propanenitrile (Preparation 3) (3.0g, 13.04mmol) in tetrahydrofuran (20ml) at -78°C and stirred for 10 minutes. Ethyl 3-bromopropionate (2.83g, 15.65mmol) was added followed by tetra-n-butylammonium iodide (150mg, 0.4mmol). The reaction mixture was then allowed to warm to room temperature and was stirred for 18 hours. A solution of ammonium acetate (1.8g) in water (10ml) was added to quench the reaction mixture, which was then extracted with ethyl acetate (x2). The combined organic layers were washed with aqueous hydrochloric acid (1N, 50ml), brine, dried over $MgSO_4$, filtered and solvent was removed under reduced pressure. The residue was purified by column chromatography on silica gel using a gradient system of hexane : diethyl ether (3 :1, by volume) changing to hexane : diethyl ether (3 : 2, by volume) to afford the title compound as an orange oil ( 2.2g, 52%). $^1$H-NMR ($CDCl_3$): $\delta$ = 7.40 (4H, m), 5.80 (1H, m), 4.10 (2H, m), 3.95 (2H, m), 3.80 (2H, m), 2.50 (2H, m), 2.30 (3H, m), 2.10 (1H, m), 1.20 (3H, m).

Preparation 5

5-(4-Chlorophenyl)-5-(1,3-dioxolan-2-ylmethyl)tetrahydro-2(1H)-pyridinone

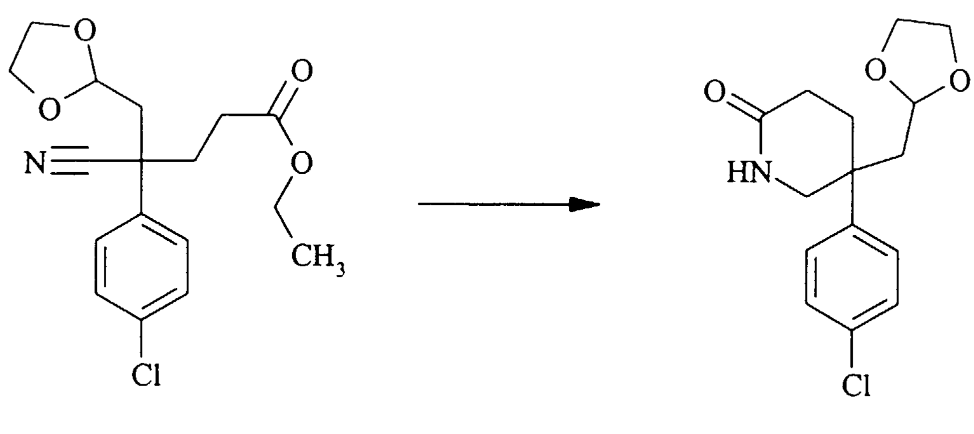

Ethyl 4-(4-chlorophenyl)-4-cyano-5-(1,3-dioxolan-2-yl)pentanoate (Preparation 4) (2g, 5.92mmol) was dissolved in glacial acetic acid (50ml), platinum oxide catalyst (0.19g) was added, the reaction mixture was pressurised to 414 kPa (60 p.s.i.) with hydrogen in a sealed vessel and the reaction mixture was stirred at room temperature for

18 hours. The reaction mixture was filtered through Arbocel (trade mark) filter aid and the solvent removed under reduced pressure. The residue was taken up in ethyl acetate and washed with saturated sodium hydrogen carbonate solution, dried over $MgSO_4$, filtered and solvent removed under reduced pressure. The residue was triturated with ethyl acetate and filtered to afford the title compound as a white solid (440mg, 25%).
$^1$H-NMR ($CDCl_3$): $\delta$ = 7.30 (4H, m), 5.95 (1H, bs), 4.35 (1H, m), 3.90 (3H, m), 3.70 (2H, m), 3.50 (1H, m), 2.40 (1H, m), 2.15 (4H, m), 1.90 (1H, m).

Preparation 6

5(S)-5-(4-Chlorophenyl)-5-(1,3-dioxolan-2-ylmethyl)tetrahydro-2(1H)-pyridinone

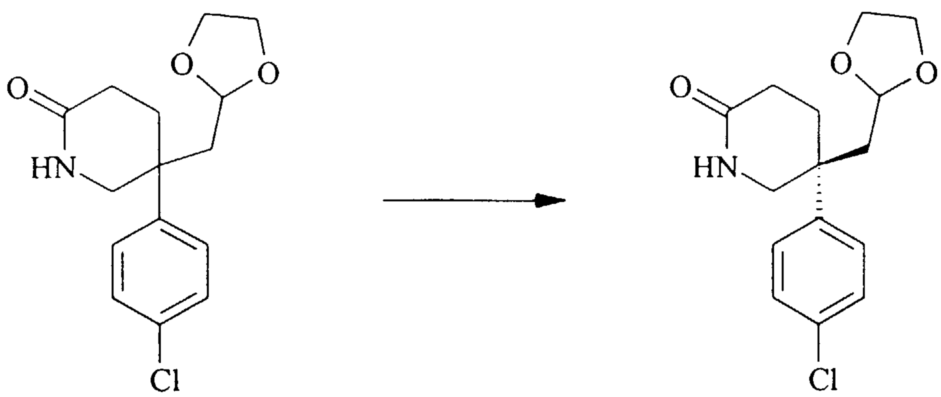

The racemic material from Preparation 5 was resolved by chiral HPLC using a Chiralpak (trade mark) AD 150 x 20mm column, eluting with 30:70, by volume, propan-2-ol:hexane at 10ml/min., detecting peaks at 214nm. Peak 2 provided the active enantiomer which was assigned the 5S stereochemistry (when compared with other compounds of known stereochemistry). $^1$H-NMR data are as for the product of Preparation 5.

Preparation 7

3(S)-3-(4-Chlorophenyl)-3-(1,3-dioxolan-2-ylmethyl)piperidine

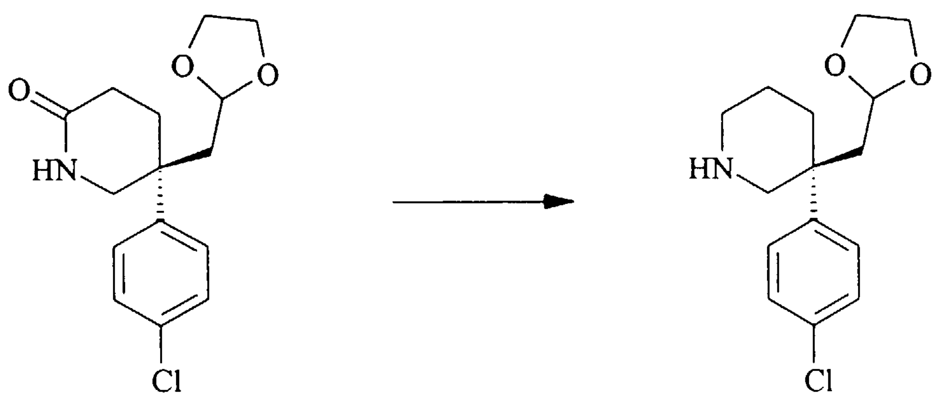

Lithium aluminium hydride (438mg, 11.5mmol) was added to a solution of 5(*S*)-5-(4-chlorophenyl)-5-(1,3-dioxolan-2-ylmethyl)tetrahydro-2(1*H*)-pyridinone (Preparation 6) (1.69g, 5.76mmol) in tetrahydrofuran (50ml) under nitrogen and the mixture stirred at room temperature for 18 hours. The reaction mixture was quenched by the addition of water (1ml) and aqueous sodium hydroxide solution (2N, 1ml), and the white precipitate was removed by filtration. The filtrate was dried over $MgSO_4$, filtered and solvent removed under reduced pressure to afford the title compound

as a colourless oil (1.6g, 99%).
$^1$H-NMR ($CDCl_3$): δ = 7.30 (4H, m), 4.35 (1H, m), 3.90 (2H, m), 3.70 (2H, m), 3.35 (1H, dd), 3.00 (1H, dd), 2.80 (2H, m), 2.15 (1H, m), 2.00 (2H, m), 1.70-1.30 (4H, m).
m/z: 282 ($MH^+$).

Preparation 8

3(S)-1-Benzoyl-3-(4-chlorophenyl)-3-(1,3-dioxolan-2-ylmethyl)piperidine

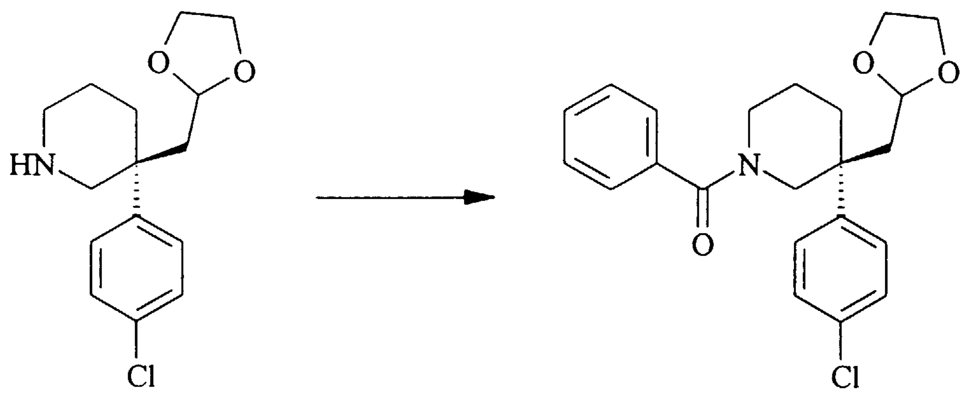

The title compound was prepared by a similar method to Preparation 1 from 3(S)-3-(4-chlorophenyl)-3-(1,3-dioxolan-2-ylmethyl)piperidine (Preparation 7) (1.6g, 5.7mmol) and benzoyl chloride (0.73ml, 6.27mmol) to afford the title compound as an off-white solid (2.03g, 92%).
$^1$H-NMR ($CDCl_3$): δ = 7.30 (6H, m), 7.20 (3H, m), 4.80 (1H, bm), 4.40 (1H, bm), 3.90 (2H, m), 3.70 (2H, m), 3.50 (1H, m), 3.30 (1H, m), 3.10 (1H, m), 2.30 (1H, m), 2.10 (1H, m), 1.90 (2H, bm), 1.50 (2H, m).
m/z: 386 ($MH^+$)

Preparation 9

3(S)-1-Benzoyl-3-(4-chlorophenyl)-3-(formylmethyl)piperidine

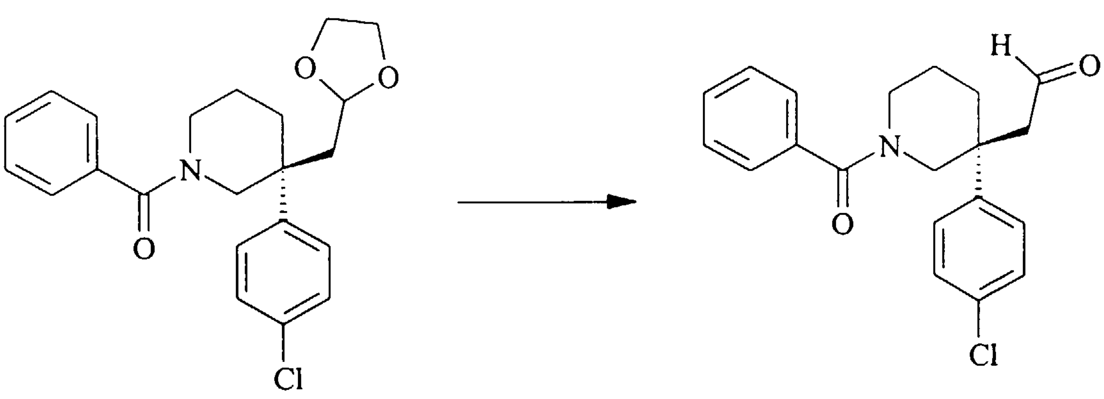

Aqueous hydrochloric acid (2N, 30ml) was added to a solution of 3(S)-1-benzoyl-3-(4-chlorophenyl)-3-(1,3-dioxolan-2-ylmethyl)piperidine (Preparation 8) (1.06g, 2.75mmol) in tetrahydrofuran (30ml) and the mixture stirred at room temperature for 18 hours. The reaction mixture was partitioned between dichloromethane and water and the layers separated. The organic layer was washed with brine, dried over $MgSO_4$ and solvent removed under reduced pressure to afford the title compound as a colourless oil (0.90g, 96%).
$^1$H-NMR ($CDCl_3$): δ = 9.50 (1H, bs), 7.40 (6H, m), 7.30 (3H, m), 4.20 (1H, bm), 3.85 (1H, m), 3.40 (2H, bm), 2.70 (2H,

bm), 2.25 (1H, m), 2.05 (1H, m), 1.60 (2H, bm).
m/z: 342 ($MH^+$).

Preparation 10

4-Cyano-4-(3,4-difluorophenyl)-5-(1,3-dioxolan-2-yl)pentanoic acid

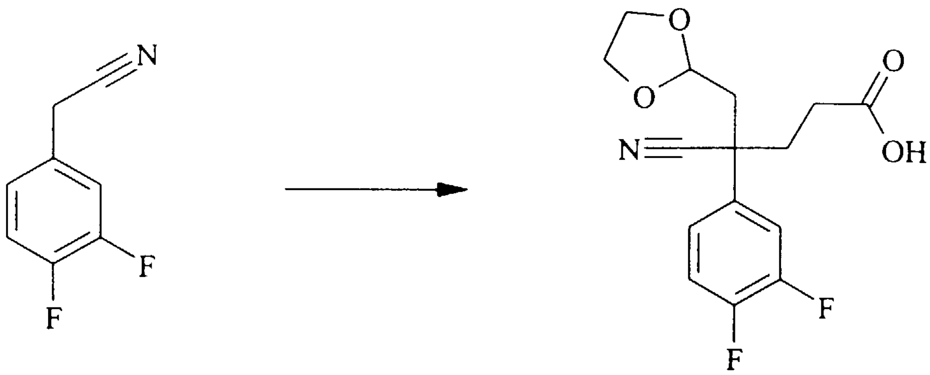

A solution of 3,4-difluorobenzyl cyanide (20g, 0.13mol) in tetrahydrofuran (20ml) was added dropwise to a solution of lithium bis(trimethylsilyl)amide (1.0M, 144ml, 0,14mol) in tetrahydrofuran at 0°C under nitrogen. The reaction mixture was allowed to warm to room temperature and stirred for 2 hours, after which time it was cooled to 0°C and a solution of 2-bromomethyl-1,3-dioxolane (15ml, 0.14mol) in tetrahydrofuran (15ml) was added followed by tetra-n-butylammonium iodide (2g, 5.4mmol). The reaction mixture was warmed to room temperature and stirred for 18 hours.

A further portion of solution of lithium bis(trimethylsilyl)amide in tetrahydrofuran (1M, 144ml, 0.14mol) was added dropwise to the reaction mixture at 0°C and then the mixture was allowed to warm to room temperature over 5 hours. The reaction mixture was cooled to 0°C and a solution of ethyl 3-bromopropionate (18ml, 0.14mol) in tetrahydrofuran (20ml) was added dropwise. The reaction mixture was allowed to warm to room temperature and stirred for 18 hours.

A solution of sodium hydroxide (7.8g, 0.20mol) in water (50ml) was added to the crude reaction mixture at 0°C and the mixture then allowed to warm to room temperature. The reaction mixture was stirred for 36 hours. The reaction mixture was partitioned between water and diethyl ether, the organic layer re-extracted with water and the combined aqueous layers were acidified to pH 1.0 with aqueous hydrochloric acid (2N). The aqueous layer was extracted with ethyl acetate (x2), dried over $Na_2SO_4$ and the solvent removed under reduced pressure. The residue was purified by column chromatography on silica gel eluting with a gradient system of dichloromethane gradually changing to dichloromethane : methanol : acetic acid (95 : 5 : 1, by volume) to afford the title compound as a brown oil (15.0g, 37%) which solidified on standing.
$^1$H-NMR ($CDCl_3$): $\delta$ = 7.30 (1H, m), 7.20 (2H, m), 4.80 (1H, m), 3.95 (2H, m), 3.80 (2H, m), 2.50 (2H, m), 2.30 (2H, m), 2.20 (2H, m).
m/z: 312 ($MH^+$).

Preparation 11

5-(3,4-Difluorophenyl)-5-(1,3-dioxolan-2-ylmethyl)tetrahydro-2(1H)-pyridinone

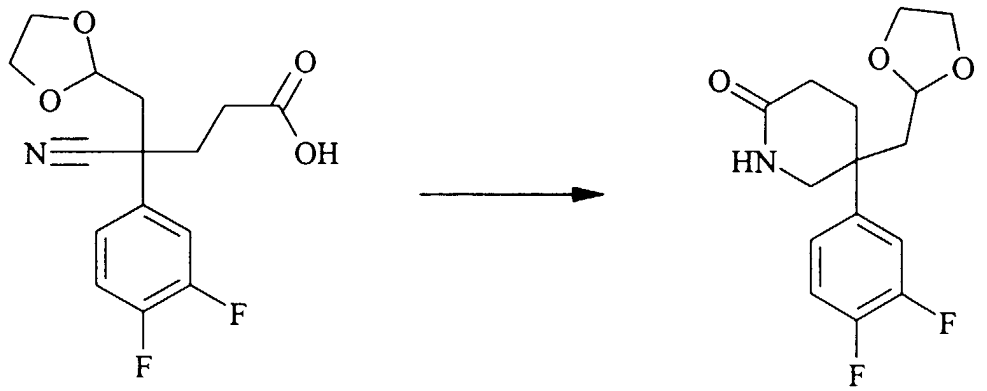

The title compound was prepared by a similar method to Preparation 5 from 4-cyano-4-(3,4-difluorophenyl)-5-(1,3-dioxolan-2-yl)pentanoic acid (Preparation 10) (7.0g, 22.5mmol). The residue was purified by column chromatography eluting with a gradient system of dichloromethane changing to dichloromethane:methanol (97 : 3, by volume) to afford the title compound as an oil (2.02g, 31%).
$^1$H-NMR ($CDCl_3$): δ = 7.20-7.00 (3H, m), 6.40 (1H, bs), 4.40 (1H, m), 3.90 (3H, m), 3.70 (2H, m), 3.50 (1H, d), 2.40 (1H, m), 2.20 (4H, m), 1.80 (1H, m). m/z: 298 ($MH^+$).

Preparation 12

5(*S*)-5-(3,4-Difluorophenyl)-5-(1,3-dioxolan-2-ylmethyl)tetrahydro-2(1H )-pyridinone

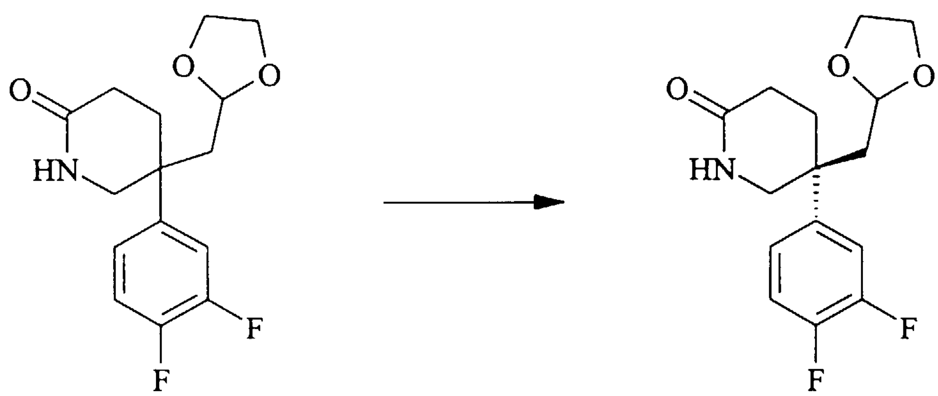

The racemic material from Preparation 11 was resolved by chiral HPLC using a Chiralpak (trade mark) AD 250 x 20mm column, eluting with 20:80, by volume, propan-2-ol:hexane at 10ml/min, detecting peaks at 220nm. Peak 1 provided the active enantiomer which was assigned the 5S stereochemistry (when compared with other compounds of known stereochemistry). $^1$H-NMR and m/z data are as for the product of Preparation 11.

Preparation 13

3(S)-3-(3,4-Difluorophenyl)-3-(1,3-dioxolan-2-ylmethyl)piperidine

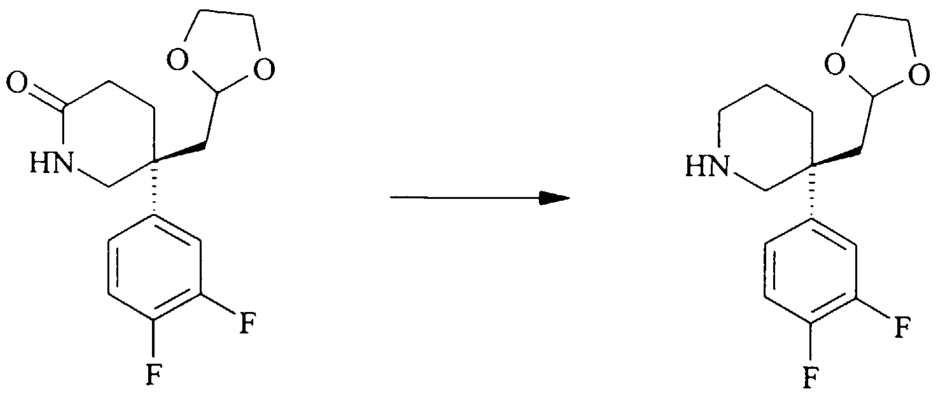

5(S)-5-(3,4-Difluorophenyl)-5-(1,3-dioxolan-2-ylmethyl)tetrahydro-2(1H)-pyridinone (Preparation 12) (565mg, 1.9mmol) was added to a solution of lithium aluminium hydride (144mg, 3.8mmol) in tetrahydrofuran (20ml) under nitrogen and the mixture stirred for 3 hours. The reaction mixture was quenched by the addition of water (1.5ml) and 2N aqueous sodium hydroxide solution (0.5ml), the white precipitate was removed by filtration and washed with tetrahydrofuran and diethyl ether. The solvent was removed from the filtrate under reduced pressure. The residue was taken up in dichloromethane, dried over $MgSO_4$, filtered and solvent removed from the filtrate under reduced pressure to afford the title compound as an oil (390mg, 72%).
$^1$H-NMR ($CDCl_3$): $\delta$ = 7.20-7.00 (3H, m), 4.40 (1H, m), 3.90 (2H, m), 3.70 (2H, m), 3.30 (1H, d), 3.00 (1H, d), 2.80 (2H, m), 2.10 (1H, m), 1.95 (2H, m), 1.80 (1H, m), 1.60 (1H, m), 1.50 (1H, m).
m/z: 284 ($MH^+$).

Preparation 14

3(S)-1-Benzoyl-3-(3,4-difluorophenyl)-3-(1,3-dioxolan-2-ylmethyl)piperidine

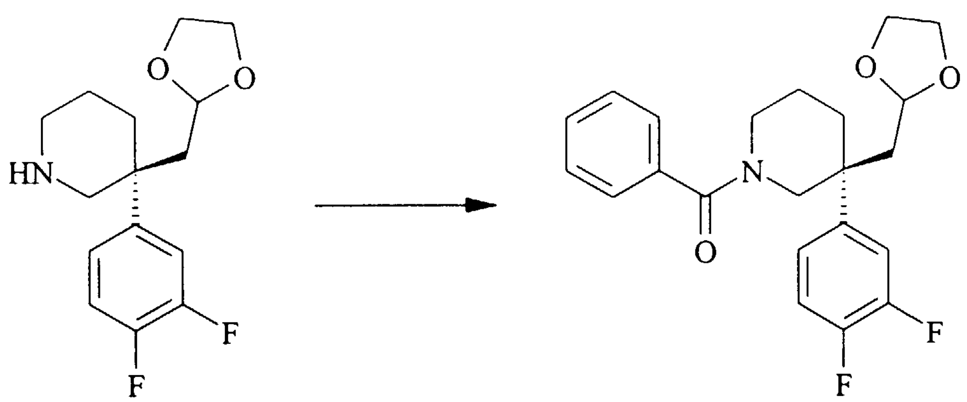

The title compound was prepared by a similar method to Preparation 1 from 3(S)-3-(3,4-difluorophenyl)-3-(1,3-dioxolan-2-ylmethyl)piperidine (Preparation 13) (377mg, 1.33mmol) and benzoyl chloride (0.28ml, 1.99mmol). The crude product was purified by column chromatography on silica gel eluting with a gradient system of dichloromethane gradually changing to dichloromethane : methanol (95 : 5, by volume) to afford the title compound as an off-white

solid (460mg, 89%).
$^1$H-NMR ($CDCl_3$): $\delta$ = 7.40 (5H, m), 7.20 (3H, m), 4.20 (1H, m), 3.90 (2H, m), 3.70 (2H, m), 3.50 (1H, d), 3.40-3.20 (1H, m), 2.30 (1H, bm), 2.20-1.80 (4H, bm), 1.80-1.40 (3H, m).
m/z: 388 ($MH^+$).

Preparation 15

3(S)-1-Benzoyl-3-(3,4-difluorophenyl)-3-(formylmethyl)piperidine

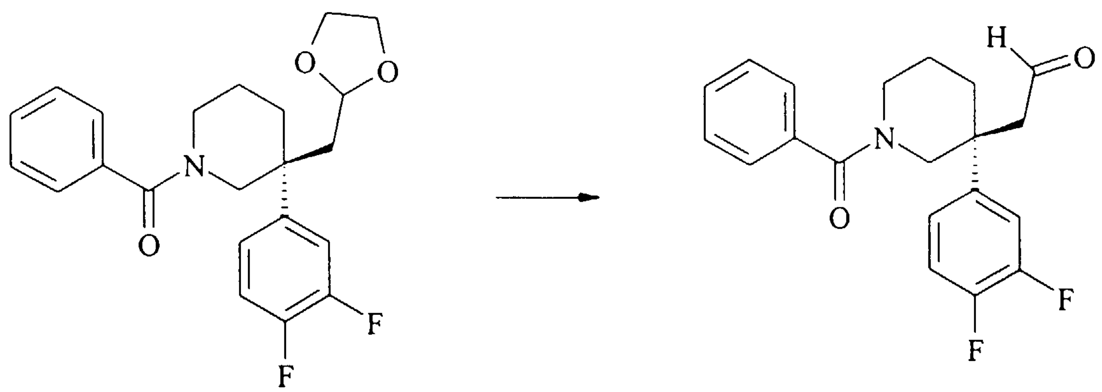

The title compound was prepared by a similar method to Preparation 2 using 3(S)-1-benzoyl-3-(3,4-difluorophenyl)-3-(1,3-dioxolan-2-ylmethyl)piperidine (Preparation 14) (450mg, 1.16mmol) and Amberlyst (trade mark) 15 ion exchange resin (500mg). The crude product was purified by column chromatography on silica gel eluting with a gradient system of dichloromethane gradually changing to dichloromethane : methanol (97 : 3, by volume) to afford the title compound as an oil (340mg, 85%).
$^1$H-NMR ($CDCl_3$): $\delta$ = 9.50 (1H, bs), 7.40 (4H, m), 7.20 (4H, m), 3.90 (1H, m), 3.55 (1H, s), 3.20 (2H, s), 2.70 (2H, bm), 2.20 (1H, m), 2.10 (1H,m), 1.60 (2H, m).
m/z: 344 ($MH^+$).

Preparation 16

Diethyl 3-(4-chlorophenyl)-3-cyano-1,5-pentanedioate

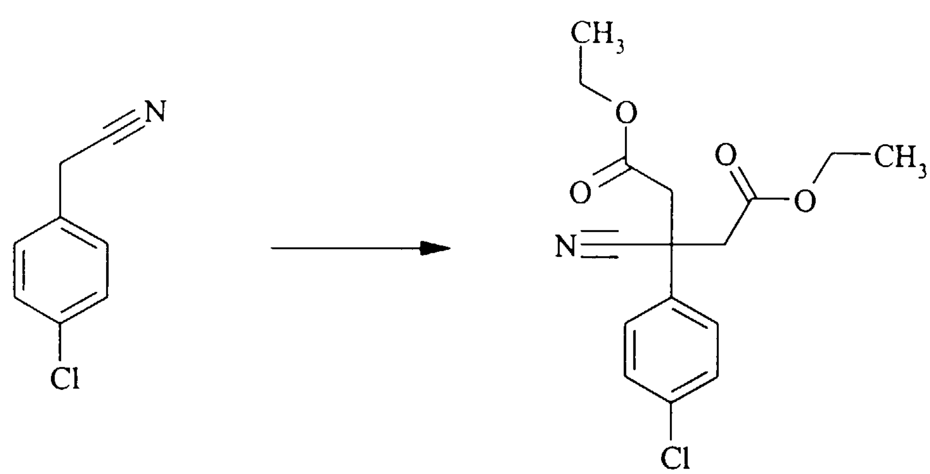

A solution of lithium bis(trimethylsilyl)amide in tetrahydrofuran (1M, 264ml, 0.26mol) was added dropwise over a period of 30 minutes to a solution of 4-chlorobenzyl cyanide (20g, 0.13mol) in tetrahydrofuran (100ml) at -78°C. The reaction mixture was allowed to warm to room temperature and stirred for 3 hours. The reaction mixture was cooled to -78°C, ethyl 2-bromoacetate (29.3ml, 0.26mol) was added and reaction mixture was allowed to warm to room temperature and stirred for 24 hours. Diethyl ether was added and reaction mixture washed with water (x2), brine, dried over $MgSO_4$, filtered and the solvent removed under reduced pressure. The residue was purified by column chromatography on silica gel eluting with a gradient system of pentane followed by diethyl ether to afford the title compound as an orange oil (37.1g, 90%).

$^1$H-NMR ($CDCl_3$): $\delta$ = 7.40 (2H, dd), 7.35 (2H, dd), 4.00 (4H, m), 3.20 (2H, dd), 3.00 (2H, dd), 1.10 (6H, m).

m/z: 341, 343 ($MNH_4^+$).

Preparation 17

Ethyl (4-(4-chlorophenyl)-2-oxopyrrolidin-4-yl)acetate

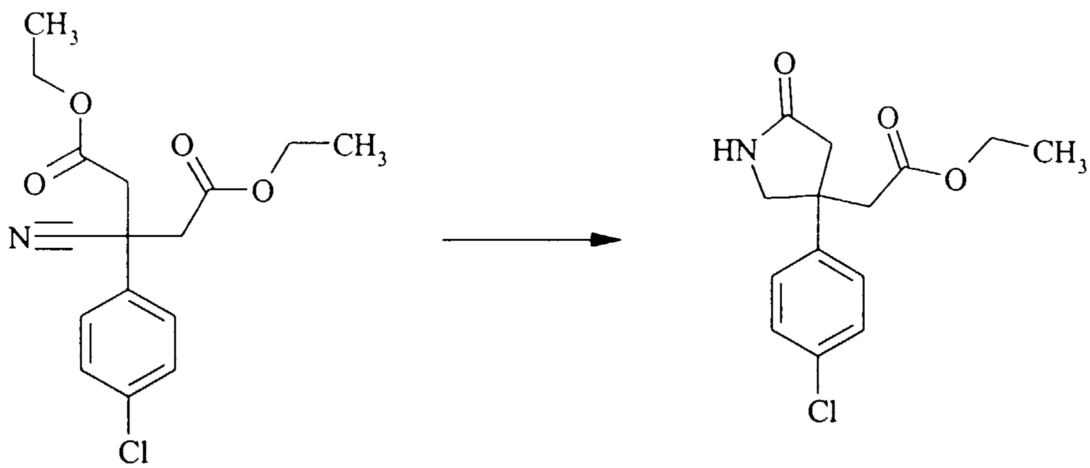

Sodium borohydride (31.75g, 0.84mol was added portionwise to a solution of diethyl 3-(4-chlorophenyl)-3-cyano-1,5-pentanedioate (Preparation 16) (26.2g, 0.08mol) and cobalt chloride (39.82g, 0.17mol) in methanol (200ml) at 0°C and the mixture stirred for 1 hour with warming to room temperature. The solvent was removed under reduced pressure and the residue was dissolved in aqueous hydrochloric acid (400ml, 1N) and dichloromethane (200ml) and stirred for 1hour. The layers were separated and organic layer was washed with brine, dried over $MgSO_4$, filtered and solvent removed under reduced pressure. The residue was purified by column chromatography on silica gel eluting with a gradient system of dichloromethane gradually changing to dichloromethane : methanol (95 : 5, by volume) to afford the title compound as a brown oil (15.9g, 70%).

$^1$H-NMR ($CDCl_3$): $\delta$ = 7.35 (2H, dd), 7.10 (2H, dd), 6.20 (1H, bs), 3.95 (3H, m), 3.70 (1H, dd), 2.80 (4H, dd), 1.10 (3H, m).

m/z: 282 ($MH^+$)

Preparation 18

2-[3-(4-Chlorophenyl)pyrrolidin-3-yl]ethanol

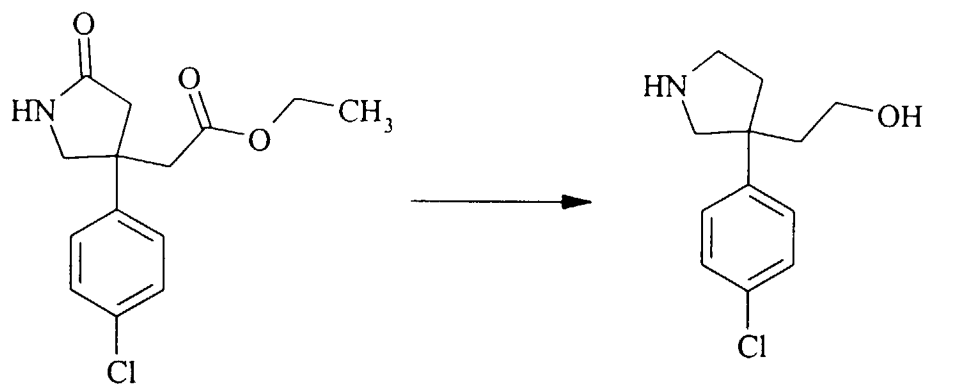

Lithium aluminium hydride (1.35g, 35.56mmol) was added to a solution of ethyl (4-(4-chlorophenyl)-2-oxopyrrolidin-4-yl)acetate (Preparation 17) (1.0g, 3.56mmol) in tetrahydrofuran (20ml) under nitrogen and the mixture stirred and heated under reflux for 18 hours. The reaction was quenched by the addition of water (7ml) and aqueous sodium hydroxide solution (2N, 5ml). The reaction mixture was diluted with dichloromethane, $MgSO_4$ was added, the solids were filtered off and solvent removed under reduced pressure to afford a solid. This solid was washed with diethyl ether (x2) and azeotroped with dichloromethane to afford the title compound as a white solid (700mg, 88%).
$^1$H-NMR ($CD_3OD$): $\delta$ = 7.30 (4H, m), 3.30 (3H, m), 3.10 (2H, m), 2.90 (1H, m), 2.10 (2H, m), 1.90 (2H, m).
m/z: 226, 228 ($MH^+$)

Preparation 19

1-Benzoyl-3-(4-chlorophenyl)-3-(2-hydroxyethyl)pyrrolidine

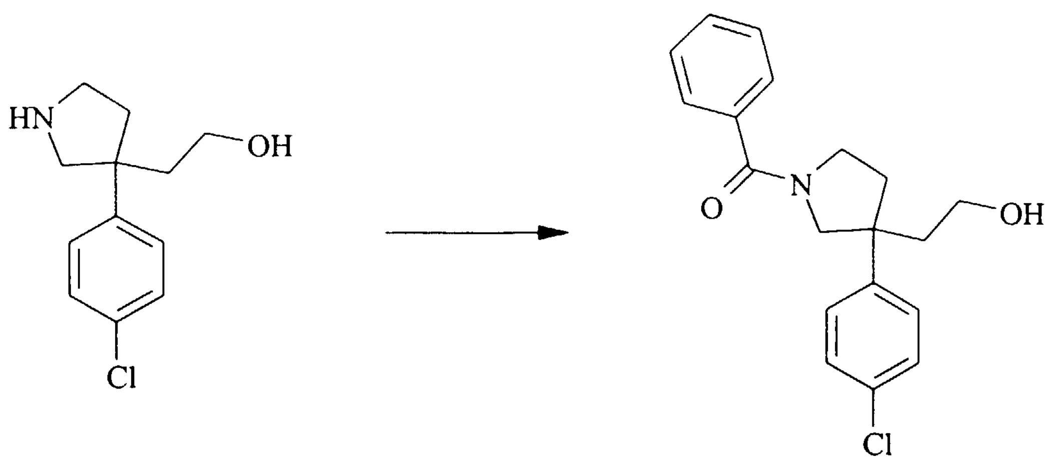

Benzoyl chloride (1.04ml, 8.94mmol) was added to a solution of 2-[3-(4-chlorophenyl)pyrrolidin-3-yl]ethanol (Preparation 18) (670mg, 2.98mmol) and triethylamine (1.66ml, 11.92mmol) in dichloromethane (30ml) at 0°C. The reaction mixture was stirred under nitrogen and warmed to room temperature over 1.5 hours. Dichloromethane was added and the reaction mixture washed with water (x2), brine, dried over $MgSO_4$, filtered and solvent removed from the filtrate under reduced pressure. The residue was taken up in ethanol and heated to 60°C. Aqueous sodium hydroxide solution (15ml, 2N) was added and the mixture stirred for 2 hours at room temperature. The ethanol was

removed under reduced pressure. Water and aqueous sodium hydroxide solution were added to the residue and the aqueous layer extracted with dichloromethane. The organic layer was washed with brine, dried over $MgSO_4$, filtered and solvent removed under reduced pressure. The crude product was purified by column chromatography on silica gel eluting sequentially with a gradient system of hexane, ethyl acetate and ethyl acetate : methanol ( 95 : 5, by volume) to afford the title compound as a colourless oil (720mg, 73%).
$^1$H-NMR ($CDCl_3$): δ = 7.40-7.20 (8H, m), 7.20-7.00 (1H, m), 4.00-3.20 (6H, m), 2.40-1.80 (4H, m).
m/z: 330, 332 ($MH^+$).

Preparation 20

2-(1-Benzoyl-3-(4-chlorophenyl)-3-pyrrolidinyl)acetaldehyde

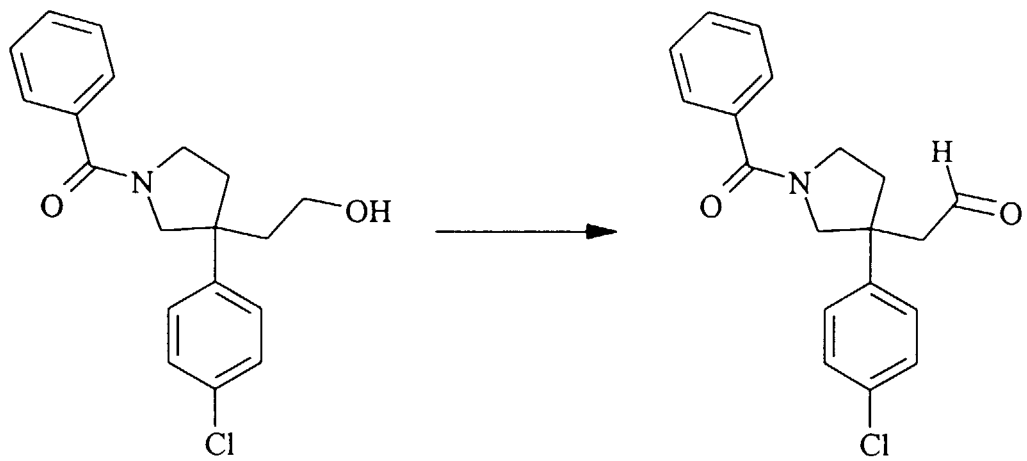

A solution of dimethylsulfoxide (0.36ml, 5.09mmol) in dichloromethane (5ml) was added to solution of oxalyl chloride (0.20ml, 2.33mmol) in dichloromethane (5ml) at -60°C and the mixture stirred for 5 minutes. A solution of 1-benzoyl-3-(4-chlorophenyl)-3-(2-hydroxyethyl)pyrrolidine (Preparation 19) (700mg, 2.12mmol) in dichloromethane (5ml) was added dropwise to the reaction mixture and the mixture stirred for 15 minutes. Triethylamine (1.48ml, 10.61mmol) was added and the reaction mixture warmed to room temperature and stirred for 3 hours. Dichloromethane was added to the reaction mixture and it was washed with aqueous hydrochloric acid (2N), brine, dried over $MgSO_4$ and solvent removed under reduced pressure to afford the title compound as a colourless oil (0.65, 93%).
$^1$H-NMR ($CDCl_3$): δ = 9.35-9.40 (1H, bm), 7.60-7.20 (8H, m), 7.20-7.00 (1H, m), 4.10 (1H, m), 3.80 (2H, m), 3.60-3.40 (1H, bm), 2.90 (1H, m), 2.80-2.65 (1H, m). 2.35 (2H, m).
$R_f$ : 0.64 (dichloromethane : methanol : 0.88 ammonia, 90 : 10 : 1, by volume).

Preparation 21

1-Benzhydryl-3-iodoazetidine

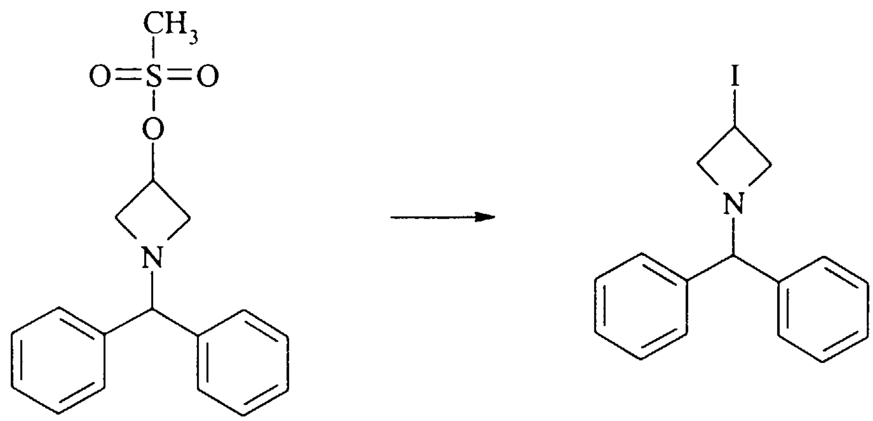

A solution of potassium iodide (60g, 0.361mol) in water (300ml) was added to a solution of 1-benzhydryl-3-methanesulphonyloxyazetidine (see WO-A-96/05193) (60g, 0.189mol) in 1,2-dimethoxyethane (600ml). The reaction mixture was heated under reflux for 2.5 hours. After this time, the reaction was cooled to room temperature and partitioned between ethyl acetate and dilute aqueous sodium carbonate solution. The organic layer was dried over $Na_2SO_4$, filtered and solvent removed under reduced pressure. The residue was purified by column chromatography on silica gel eluting with diethyl ether. The product was recrystallised from diisopropyl ether to afford the title compound (41g, 62%).
$^1$H-NMR ($CDCl_3$): $\delta$ = 7.10-7.50 (10H, m), 4.70 (1H, s), 4.40-4.50 (1H, m), 3.80-4.0 (2H, m), 3.40-3.60 (2H, m).

Preparation 22

4-(1-Benzhydrylazetidin-3-yl)pyridine

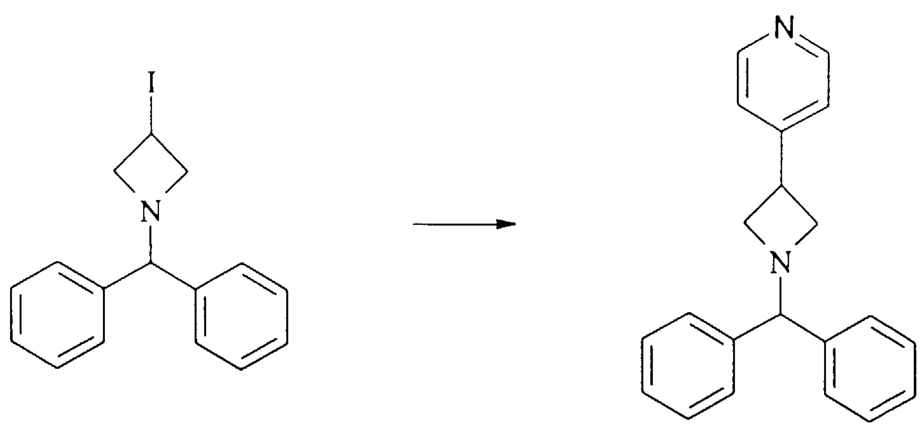

1,2-Dibromoethane (2.1 ml, 24mmol) was added to a suspension of zinc dust (17g, 0.26mol) in tetrahydrofuran (40ml) under nitrogen. The resultant mixture was heated under reflux, to initiate the reaction, for 5 minutes. After cooling to room temperature, trimethylsilyl chloride (2.5ml, 0.02mol) was added and the mixture stirred for 1 hour. A solution of 1-benzhydryl-3-iodoazetidine (Preparation 21) (70g, 0.20mol) in tetrahydrofuran (40ml) was then added portionwise. The temperature of the reaction mixture was maintained between 28-32°C during the addition using a cold water bath and the mixture then stirred at room temperature for 4 hours. 4-Chloropyridine (22.7g, 0.2mol) was added, followed by bis(dibenzylideneacetone) palladium (2.8g, 3mmol) and tri-(o-furyl)phosphine (2.4g, 10mmol), and the reaction mixture was heated under reflux for 5 hours and then stirred at room temperature for 15 hours. After this time the reaction mixture was partitioned between dilute aqueous sodium carbonate solution and ethyl acetate. The aqueous layer was extracted with ethyl acetate (×3) and the combined organic layers were dried over $Na_2SO_4$, filtered and solvent removed under reduced pressure. The residue was purified by column chromatography on silica gel eluting with a gradient system of ethyl acetate : pentane (80:20, by volume) changing to ethyl acetate to afford the title compound as a yellow oil (14g, 23%).
$^1$H-NMR ($CDCl_3$): δ = 8.50 (2H, m), 7.10-7.50 (12H, m), 4.40 (1H, s), 3.50-3.70 (3H, m), 3.10-3.20 (2H, m).

Preparation 23

4-(1-Benzhydrylazetidin-3-yl)piperidine

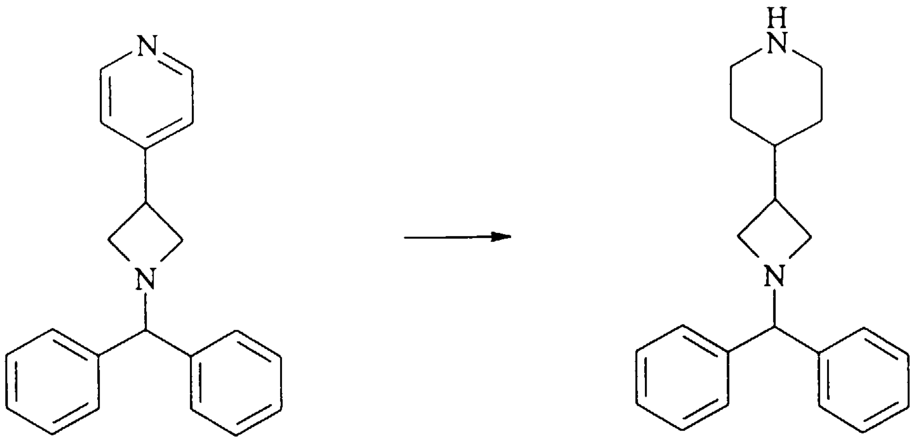

Aqueous hydrochloric acid (1.0M, 200ml) was added to a solution of 4-(1-benzhydrylazetidin-3-yl)pyridine (Preparation 22) (29.7g, 99mmol) in methanol (300ml) and methanol (100ml) and water (100ml) then added. Platinum oxide (5.0g) was added and the reaction mixture was hydrogenated for 18 hours at 50°C and a pressure of 414 kPa (60 p.s.i.). After this time, the reaction mixture was filtered through Arbocel (trade mark) filter aid and the solvent was removed under reduced pressure. A solution of sodium hydroxide (25g) in water (200ml) was added to the residue and the resultant mixture was extracted with diethyl ether (x3). The combined organic layers were dried over $Na_2SO_4$, filtered and solvent removed under reduced pressure to produce a solid. This solid was triturated with cold diisopropyl ether (50ml) to afford the title compound as a solid (22.7g, 75%).
$^1$H-NMR ($CDCl_3$): δ = 7.10-7.40 (10H, m), 4.30 (1H, s), 3.20-3.40 (2H, m), 2.90-3.10 (2H, m), 2.60-2.80 (2H, m), 2.40-2.60 (2H, m), 2.10-2.30 (1H, m), 1.30-1.80 (4H, m), 0.80-1.00 (2H, m).

Preparation 24

4-(1-Benzhydrylazetidin-3-yl)-1-(methylsulfonyl)piperidine

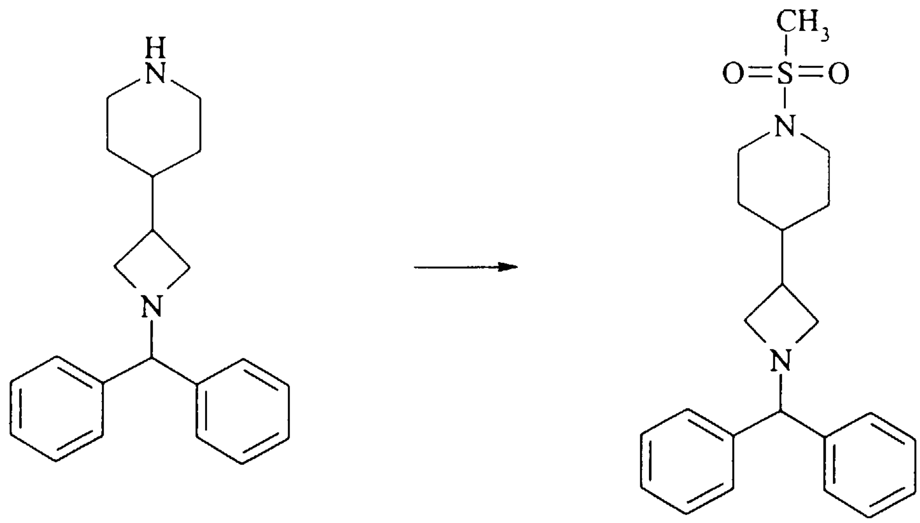

Triethylamine (14ml, 0.1mol) was added to a solution of 4-(1-benzhydrylazetidin-3-yl)piperidine (Preparation 23) (22g, 72mmol) in dry dichloromethane (200ml) at 0°C. Methanesulfonyl chloride (6.2ml, 80mmol) was added dropwise and the reaction mixture was stirred for 15 hours, after which time further triethylamine (5ml) and methanesulfonyl chloride (0.7ml) were added. The reaction mixture stirred for 2 hours. The reaction mixture was diluted with dichloromethane and washed with dilute aqueous sodium hydroxide solution. The organic layer was dried over $Na_2SO_4$, filtered and solvent removed under reduced pressure. The residue was purified by column chromatography on silica gel, eluting with a gradient system of ethyl acetate : pentane : dichloromethane (40:40:20, by volume) changing to ethyl acetate : dichloromethane (50:50, by volume) to give the title compound as a solid (15.1g, 55%).
$^1H$-NMR ($CDCl_3$): δ = 7.10-7.40 (10H, m), 4.30 (1H, s), 3.70-3.80 (2H, m), 3.20-3.40 (2H, m), 2.70-2.80 (5H, m), 2.50-2.70 (2H, m), 2.10-2.30 (1H, m), 1.60-1.80 (2H, m), 1.40-1.60 (1H, m), 1.10-1.30 (2H, m).

Preparation 25

4-(Azetidin-3-yl)-1-(methylsulfonyl)piperidine

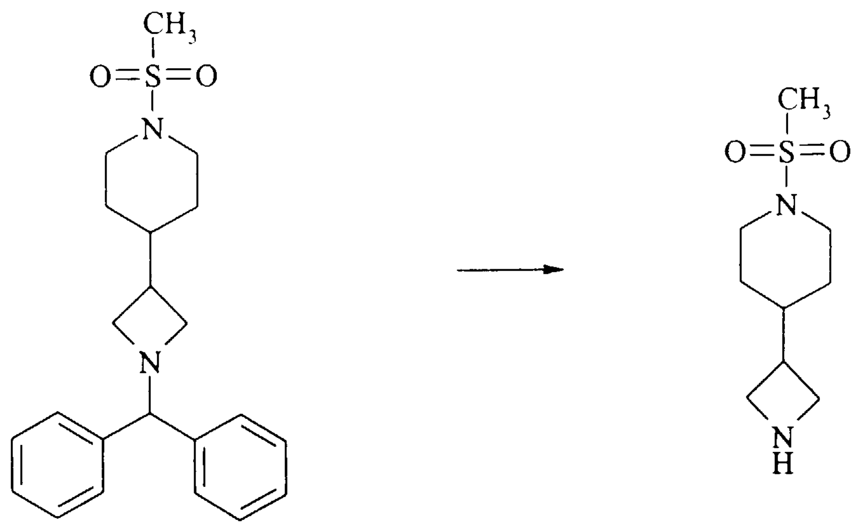

Aqueous hydrochloric acid (2.0M, 25ml) was added to a solution of 4-(1-benzhydrylazetidin-3-yl)-1-(methylsulfonyl)piperidine (15.0g, 39mmol) (Preparation 24) in methanol (400ml). Palladium hydroxide (2.0g) was added and the mixture was hydrogenated for 5 hours at 60°C and at a pressure of 414 kPa (60 p.s.i.). The reaction was filtered through Arbocel (trade mark) filter aid and the solvent was removed from the filtrate under reduced pressure to remove most of the water. The residue was then partitioned between dichloromethane and aqueous sodium hydroxide solution (1M). The organic layer was dried over $Na_2SO_4$, filtered and solvent removed under reduced pressure. The residue was triturated with cold diethyl ether (200ml) to afford the title compound as a solid (6.8g, 80%).
$^1$H-NMR ($CDCl_3$): δ = 3.70-3.90 (2H, m), 3.60-3.70 (2H, m), 3.30-3.50 (2H, m), 2.80 (3H, s), 2.50-2.70 (2H, m), 2.40-2.50 (1H, m), 2.00-2.10 (1H, s), 1.70-1.80 (2H, m), 1.50-1.70 (1H, m), 1.10-1.30 (2H, m).

Preparation 26

4-(Piperidin-4-yl)pyridine

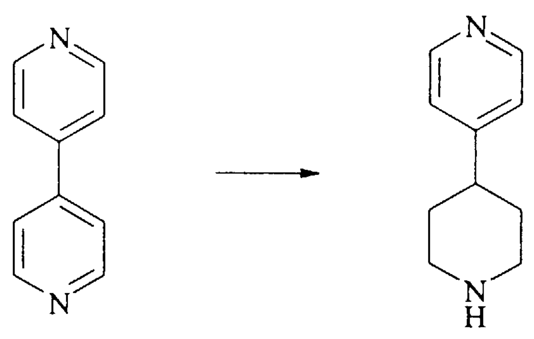

The title compound was prepared by a similar method to Preparation 23 from 4,4'-bipyridine (10g, 64mmol), platinum oxide (1.0g) and aqueous hydrochloric acid (1M, 80ml) to afford the title compound as a solid (3.41g, 33%).
$^1$H-NMR ($CDCl_3$): δ = 8.50 (2H, m), 7.10 (2H, m), 3.20 (2H, m), 2.80 (2H, m), 2.60 (1H, m), 1.80 (2H, m), 1.60 (3H, m).
m/z: 163 ($MH^+$).

Preparation 27

1-(Methylsulfonyl)-4-(pyridin-4-yl)piperidine

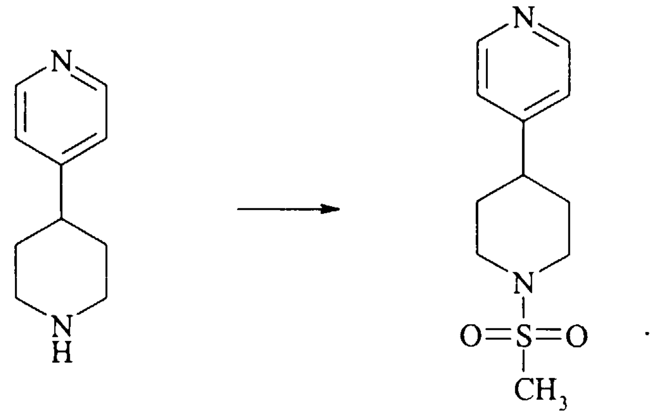

Methanesulfonyl chloride (0.98ml, 13mmol) was added dropwise to a solution of 4-(piperidin-4-yl)pyridine (Preparation 26) (1.71g, 10.5mmol) and triethylamine (2.2ml, 16mmol) in dry dichloromethane (20ml) at 0°C. The reaction mixture was stirred for 15 hours at room temperature. The reaction mixture was diluted with dichloromethane and washed with water and brine, dried over $MgSO_4$, filtered and solvent removed from filtrate under reduced pressure to afford the title compound as a solid (2.19g, 55%).
$^1$H-NMR ($CDCl_3$): $\delta$ = 8.50 (2H, m), 7.10 (2H, m), 4.00 (2H, m), 2.80 (5H, m), 2.60 (1H, m), 2.00 (2H, m), 1.85 (2H, m).
m/z: 241 ($MH^+$).

Preparation 28

1-Methylsulfonyl-4-(piperidin-4-yl)piperidine

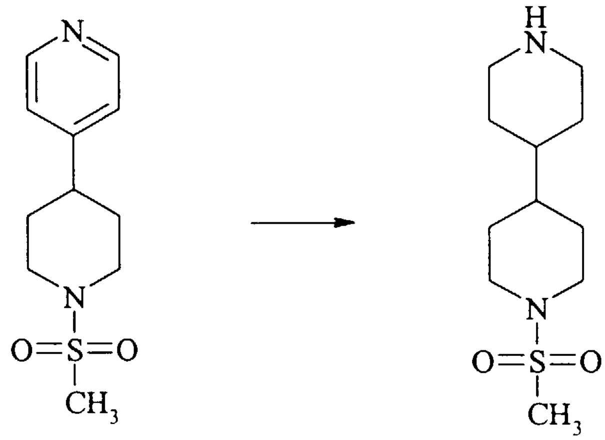

The title compound was prepared by a similar method to Preparation 23 from 1-(methylsulfonyl)-4-(pyridin-4-yl)piperidine (Preparation 27) (2.18g, 9.07mmol), platinum oxide (200mg) and aqueous hydrochloric acid (1M, 10ml) to afford the title compound as a white solid (1.90g, 85%).
$^1$H-NMR ($CDCl_3$): $\delta$ = 3.80 (2H, m), 3.10 (2H, m), 2.70 (3H, s), 2.50 (4H, m), 1.80 (2H, m), 1.60 (2H, m), 1.30 (2H, m),

1.10 (4H, m).
m/z: 247 ($MH^+$).

Preparation 29

4-(Tetrahydropyran-4-yl)piperidine

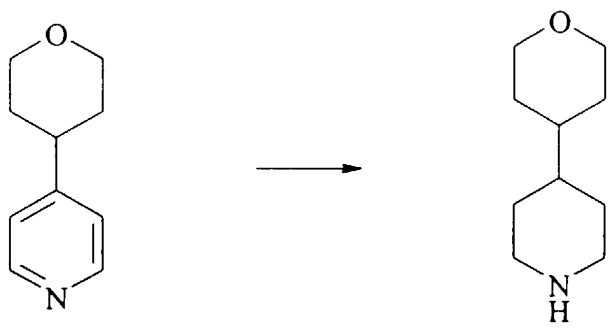

The title compound was prepared by a similar method to Preparation 23 from 4-(tetrahydropyran-4-yl)pyridine (5g, 31mmol), platinum oxide (700mg) and aqueous hydrochloric acid (1N, 35ml) to afford the title compound as a solid (5g, 97%).
$^1$H-NMR ($CDCl_3$): δ = 4.00 (2H, m), 3.30 (2H, m), 3.10 (2H, m), 2.50 (2H, m), 1.70 (3H, m), 1.60 (2H, m), 1.30 (3H, m), 1.10 (3H, m).
m/z: 170 ($MH^+$).

Preparation 30

3-Methanesulphonyloxyazetidine hydrochloride

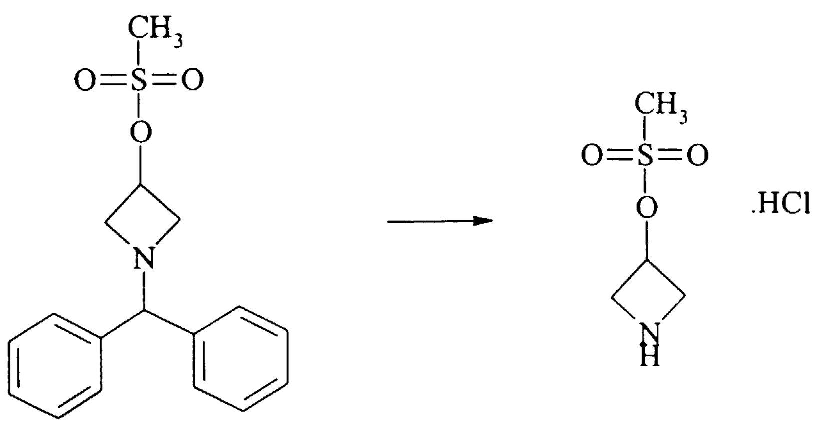

1-Benzhydryl-3-methanesulphonyloxyazetidine (40g, 126mmol) (WO-A-96/05193) was dissolved in 1,2-dichloroethane (300ml), 1-chloroethyl chloroformate (21.7ml, 200mmol) was added over 5 minutes, and the reaction stirred and heated under reflux for 2 hours. The reaction mixture was cooled, the solvent removed under reduced pressure, the residue dissolved in methanol (300ml) and stirred and heated under reflux for a further 1.25 hours. The reaction mixture was cooled, the solvent removed under reduced pressure and residue triturated with ice-cold ethyl acetate (150ml) to afford the title compound as a white solid (19.6g, 83%).
$^1$H-NMR ($d_6$-DMSO): δ = 10.00-9.40 (2H, bs), 5.30 (1H, m), 4.30 (2H, m), 4.10 (2H, m), 3.30 (3H, s).

Preparation 31

1-*tert*-Butyloxycarbonyl-3-(methylsulfonyloxy)azetidine

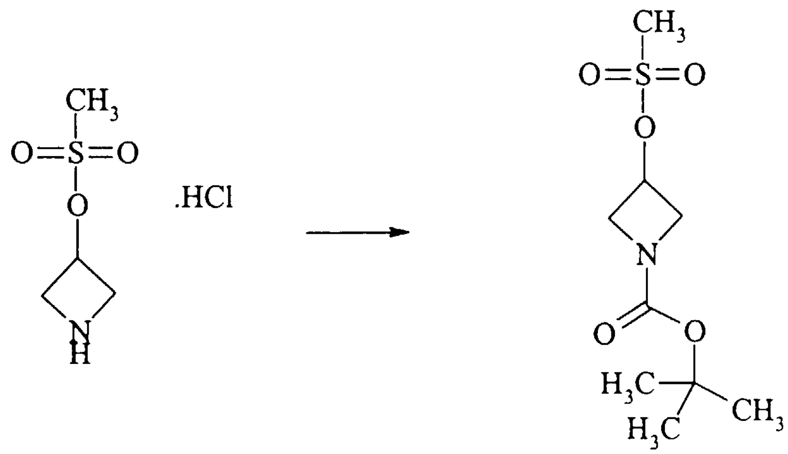

3-Methanesulphonyloxyazetidine hydrochloride (Preparation 30) (19.4g, 103mmol) was dissolved in dichloromethane (200ml), cooled to 0°C and a solution of di-t-butyl dicarbonate (22.6g, 103mmol) in dichloromethane (200ml) was added over 30 minutes. Triethylamine (30ml, 215mmol) was added dropwise over 30 minutes and reaction mixture stirred for 1 hour at 0°C then warmed to room temperature and stirred for a further 2 hours. The solution was cooled to 0°C and washed with cold aqueous hydrochloric acid (i.e. a solution of 25ml conc. hydrochloric acid in 200ml water), water, dried over $Na_2SO_4$, filtered and solvent removed under reduced pressure to give the title compound as an oil (28g).
$^1$H-NMR ($CDCl_3$): $\delta$ = 5.20 (1H, m), 4.30 (2H, m), 4.10 (2H, m), 3.05 (3H, s), 1.45 (9H, s).

Preparation 32

1-*tert*-Butyloxycarbonyl-3-iodoazetidine

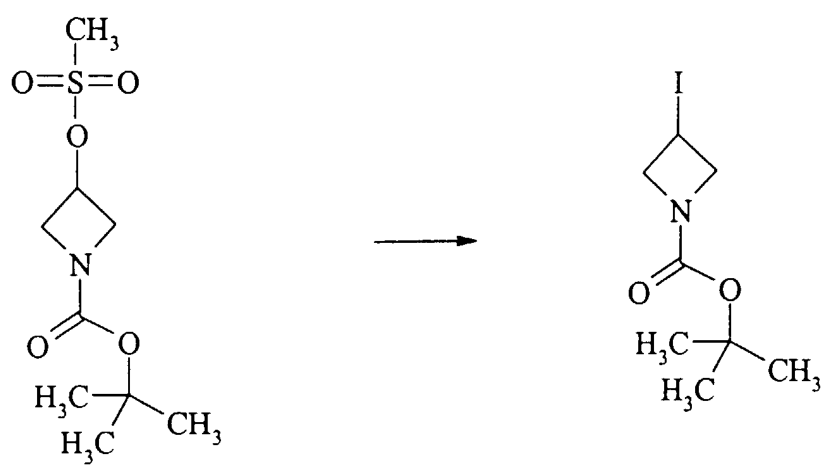

A mixture of 1-*tert*-butyloxycarbonyl-3-(methylsulfonyloxy)azetidine (Preparation 31) (28g, 111mmol) and potassium iodide (170g, 1.02mol) in dimethylsulphoxide (250ml) was heated to 140°C and stirred for 2 hours. The reaction mixture was cooled, poured into water (1000ml) and extracted with diethyl ether (x2). The combined organic layers were washed with an aqueous solution of sodium metabisulfite, brine, dried over $Na_2SO_4$, filtered and solvent removed under reduced pressure. The residue was purified by column chromatography on silica gel, eluting with a solvent

system of ethyl acetate : pentane (1:1, by volume) to give the title compound as a pale yellow oil (19g, 60%).
$^1$H-NMR ($CDCl_3$): δ = 4.65 (2H, m), 4.50 (1H, m), 4.30 (2H, m), 1.45 (9H, s).

Preparation 33

1-*tert*-Butyloxycarbonyl-3-(pyridin-4-yl)azetidine

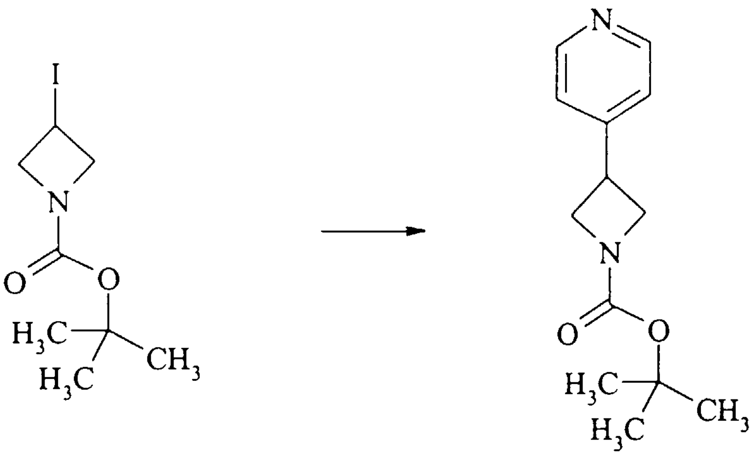

1,2-Dibromoethane (0.07ml, 0.81mmol) was added to a suspension of zinc dust (590mg, 9.07mmol) in tetrahydrofuran (2ml) under nitrogen. The resultant mixture was heated under reflux, to initiate the reaction, for 2 minutes. The reaction mixture was cooled to room temperature then heated under reflux again. After cooling to room temperature, trimethylsilyl chloride (0.10ml, 0.8mmol) was added and the mixture stirred for 30 minutes. A solution of 1-*tert*-butyloxycarbonyl-3-iodoazetidine (Preparation 32) (2.00g, 7.06mmol) in tetrahydrofuran (5ml) was added portionwise. The temperature of the reaction mixture was maintained between 28-32°C during the addition using a cold water bath and then it was stirred at room temperature for 4 hours. A solution of 4-bromopyridine (1.34g, 8.5mmol, obtained from the HCl salt by partitioning the salt between diethyl ether and aqueous sodium hydroxide solution, separation of the organic layer and removal of the solvent therefrom) in tetrahydrofuran (10ml) was added, followed by bis(dibenzylideneacetone) palladium (80mg, 0.09mmol) and tri-(o-furyl)phosphine (65mg, 0.28mmol), and the reaction mixture was stirred at room temperature for 18 hours. After this time, the reaction mixture was partitioned between ethyl acetate and a mixture of ethylenediaminetetraacetic acid (EDTA) (3.8g), sodium hydroxide (1g) and water (100 ml) (pH12-14). The aqueous layer was extracted with ethyl acetate (x3), the combined organic layers were dried over $Na_2SO_4$, filtered and solvent removed from the filtrate under reduced pressure. The residue was purified by column chromatography on silica gel eluting with a gradient system of dichloromethane : ethyl acetate (1:1, by volume) gradually changing to ethyl acetate then to ethyl acetate : methanol (90:10, by volume) to afford the title compound (900mg, 55%).
$^1$H-NMR ($CDCl_3$): δ = 8.60 (2H, m), 7.25 (2H, m), 4.35 (2H, m), 3.95 (2H, m), 3.70 (1H, m), 1.45 (9H, s).

Preparation 34

4-(Azetidin-3-yl)pyridine hydrochloride

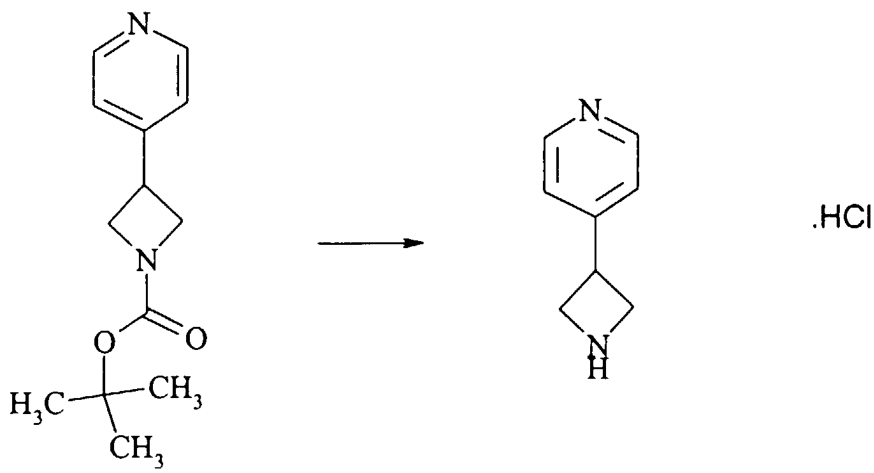

Hydrogen chloride gas was bubbled through an ice-cold, stirred solution of 1-*tert*-butyloxycarbonyl-3-(pyridin-4-yl)azetidine (Preparation 33) (1.10g, 4.70mmol) in dichloromethane (20ml) until the solution was saturated with the gas (solution became cloudy). The solvent was removed under reduced pressure, the residue dissolved in dichloromethane and the solvent was removed under reduced pressure (this procedure was repeated twice). The residue was triturated with diethyl ether (x2), filtered and dried under reduced pressure to afford the title compound as a solid (970mg, 99%).
$^1$H-NMR ($d_6$DMSO) δ = 8.90 (2H, d), 8.10 (2H, d), 4.45-4.00 (6H, m).

Preparation 35

4-[1-Methylsulfonylazetidin-3-yl]pyridine

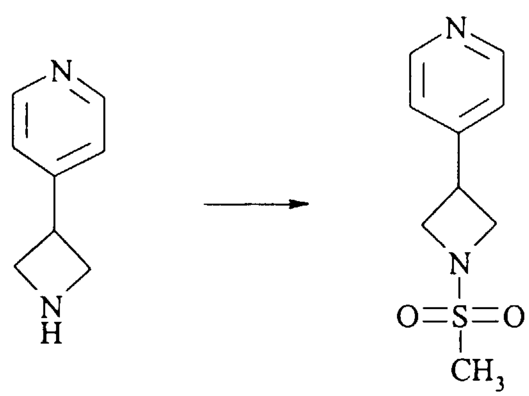

Methanesulfonyl chloride (0.4ml, 5.15mmol) was added dropwise to an ice-cooled solution of 4-(azetidin-3-yl) pyridine (Preparation 34) (970mg, 4.68mmol) and triethylamine (2.3ml, 16.38mmol) in dichloromethane (20ml). The reaction mixture was stirred for 72 hours at room temperature. The reaction mixture was washed with water and brine, dried over $MgSO_4$, filtered and solvent removed from the filtrate under reduced pressure. The residue was purified by column chromatography on silica gel, eluting with a gradient system of dich0loromethane gradually changing to dichloromethane : methanol (96:4, by volume) to give the title compound as a solid (595mg, 60%).
$^1$H-NMR ($CDCl_3$) δ: 8.60 (2H, d), 7.25 (2H, d), 4.30 (2H, t), 4.05 (2H, t), 3.80 (1H, m), 2.90 (3H, s).
m/z: 213 ($MH^+$).

Preparation 36

4-[1-Methylsulfonylazetidin-3-yl]piperidine

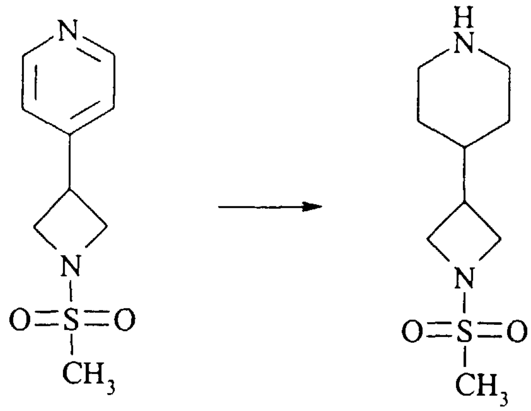

The title compound was prepared by a similar method to Preparation 23 from 4-[1-(methylsulfonyl)azetidin-3-yl]pyridine (Preparation 35) (580mg, 2.73mmol), platinum oxide (100mg) and aqueous hydrochloric acid (2N, 5ml) to afford the title compound as a solid (500mg, 84%).
$^1$H-NMR ($CDCl_3$) δ: 3.90 (2H, d), 3.70 (2H, d), 3.10 (2H, t), 2.85 (2H, t), 2.60 (1H, m), 2.35 (3H, s), 2.15 (1H, bs), 1.60 (3H, m), 1.05 (2H, m).
m/z: 219 ($MH^+$)

PHARMACOLOGICAL ACTIVITY

a) $NK_2$receptor affinity and $NK_3$ receptor affinity

Human $NK_2$ receptor affinity was tested in vitro by testing the ability to compete with [$^3$H]-NKA for binding to membranes prepared from Chinese hamster ovary cells expressing the cloned human $NK_2$ receptor using the method set out on page 10. The $pK_i$ was then determined.
$NK_3$ receptor affinity was tested, in vitro, by the method described on pages 10 and 11 by testing the ability to inhibit [$^3$H]-senktide binding to membranes from guinea pig cortex using the method of Guard, et al, Br.J.Pharmacol., 99, 767-773 (1990). The $pK_i$ was then determined.
The "$pK_i$" measurement is the negative logarithm of the molar affinity of the test compound for the receptor as determined in radioligand binding assays using standard protocols. A 1.0 log. unit difference in either the $NK_2$ or $NK_3$ receptor affinity figures corresponds to a 10-fold activity difference.

b) Metabolic stability

The metabolic stability of the compounds was determined in vitro by the method described on page 11 according to the following experimental protocol.

(i) Preparation of hepatic microsomes (HLM/37)

Transplant-quality human liver tissue was obtained from the International Institute for the Advancement of Medicine (Exton, PA). The donors ranged in age from 26 to 65 years old and included 3 males and 3 females. Hepatic microsomes were prepared from individual human livers by the process of differential centrifugation. Briefly, the liver tissue was homogenised in 50mM Tris HCl (pH 7.4) containing 250mM sucrose and then centrifuged at 9,000G for 20 minutes to remove the cell debris and nuclear fraction. The supernatant layer was removed and further centrifuged at 105,000G for 60 minutes to pellet the microsomal fraction. This pellet was washed with 100mM Tris HCl (pH 7.4) and centrifuged at 105,000G for 60 minutes to remove any contaminating haemoglobin. The final pellet was re-suspended in 100mM potassium phosphate (pH 7.4) and stored at -80°C prior to use. The cytochrome P450 content was determined using the method of Omura, T., Sato, R., J. Biol. Chem., 239, 2379-2385 (1964) and the protein concentration

was determined using the method of Lowry *et al*., J. Biol. Chem., 193, 265-275 (1951), with bovine serum albumin as the protein standard.

The metabolic activities of the 6 major drug metabolising cytochrome P450 enzymes were determined for HLM/37 and compared to values obtained from a bank of individual human livers (n = 19).

| Cytochrome P450 | Lowest Activity | Highest Activity | Mean Activity | HLM/37 Activity |
|---|---|---|---|---|
| CYP1A2 | 3.6 | 37.9 | 7.3 | 6.1 |
| CYP2C9 | 536 | 7718 | 2100 | 2733 |
| CYP2C19 | 2.7 | 133.7 | 25.9 | 25.2 |
| CYP2D6 | 2.3 | 54.8 | 18.8 | 17.1 |
| CYP2E1 | 0.2 | 2.4 | 1.2 | 1.3 |
| CYP3A4 | 69 | 6618 | 1348 | 1528 |

Based on this analysis HLM/37 appears to represent a "mean" human liver microsomal preparation.

<u>(ii) Incubations to determine disappearance half-life values</u>

Each incubation (final volume 1.2ml) was comprised of microsomal protein (equivalent to 0.5μM cytochrome P450), 50mM Tris HCl (pH 7.4), 5mM $MgCl_2$ and 5μM $MnCl_2$. Reducing equivalents required for cytochrome P450 metabolism were provided by NADPH (1 mM) that was regenerated <u>in situ</u> by an isocitric acid (5mM)/isocitric acid dehydrogenase (1 unit/ml) system (n.b. 1 unit/ml means that each ml of the incubation mixture contains 1 unit of isocitric acid dehydrogenase where 1 unit of this enzyme is defined as the amount of enzyme required to convert 1.0 micromole of isocitrate to alpha-ketoglutarate per min. at pH 7.4 and 37°C). The incubation mixture was pre-incubated at 37°C in the presence of the test compound (1μM) prior to addition of NADPH to initiate the reaction.

Aliquots (100μl) were removed from the incubation at 0, 3, 5, 10, 15, 20, 30, 45 and 60 minutes after the addition of NADPH. The reaction was terminated by immersion in ice-cold methanol (100μl). The resulting samples were centrifuged at 12,500 rpm for 5 minutes. After centrifugation 150μl aliquots were removed and 120μl of each of these analysed by HPLC on a Hypersil HS C18 5μ column (50x4.6mm) with a mobile phase of methanol:water (90:10, by volume) containing 2mM ammonium acetate. Detection was by mass spectrometry using a Sciex API-100 single quadropole mass spectrometer monitoring the protonated molecular ion ($MH^+$) of the test compound. Analysis of the chromatograms was carried out using MacQuan 1.5.

The disappearance half-life for each incubation of test compound was obtained by plotting the natural log of the test compound peak area versus time. The slope of the line of best fit through the points yields the rate of metabolism (k). This was converted to a half-life using the following relationship:

$$\text{Half life}(t_{1/2}) = \frac{\ln 2}{k}$$

<u>c) Results</u>

The compounds of Examples 2, 6 and 10 were tested for $NK_2$ and $NK_3$ receptor affinity and metabolic stability and the results obtained are presented in Table 1.

TABLE 1

| | $pK_i$ ($NK_2$) | $pK_i$ ($NK_3$) | $t_{1/2}$ (min) |
|---|---|---|---|
| Example 2 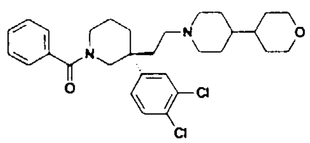 | 8.2 | 7.2 | 28 |
| Example 6 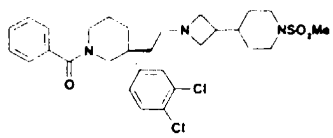 | 8.3 | 7.4 | 53 |
| Example 10 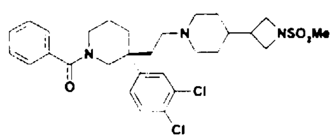 | 8.3 | 7.7 | 32 |

These data show that the compounds are potent and selective $NK_2$ receptor antagonists.

**Claims**

**1.** A compound of the formula:-

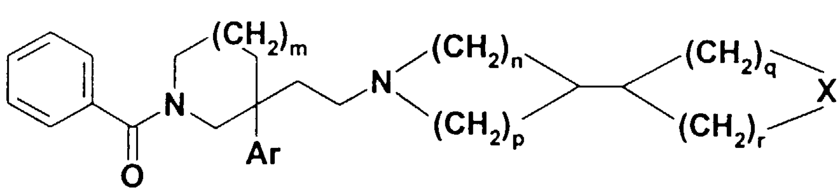

**(I)**

or a pharmaceutically acceptable acid addition salt thereof,
wherein

Ar is phenyl substituted by 1 or 2 substituent(s) each independently selected from fluoro and chloro;
X is $NSO_2(C_1\text{-}C_4$ alkyl), $NSO_2$(halo($C_1\text{-}C_4$ alkyl)) or O;
m is 0 or 1 ;
n is 1 or 2;
p is 1 or 2;
q is 1 or 2; and
r is 1 or 2.

2. A compound as claimed in claim 1 wherein Ar is 4-chlorophenyl, 3,4-dichlorophenyl, 3,4-difluorophenyl or 4-chloro-3-fluorophenyl.

3. A compound as claimed in claim 1 or 2 wherein X is $NSO_2(C_1-C_4$ alkyl) or O.

4. A compound as claimed in claim 1, 2 or 3 wherein X is $NSO_2CH_3$ or O.

5. A compound as claimed in claim 1, 2, 3 or 4 wherein n is 1 and p is 1.

6. A compound as claimed in claim 1, 2, 3 or 4 wherein n is 2 and p is 2.

7. A compound as claimed in any one of the preceding claims wherein q is 1 and r is 1.

8. A compound as claimed in any one of claims 1 to 6 wherein q is 2 and r is 2.

9. A compound as claimed in claim 1 or 2 wherein the group

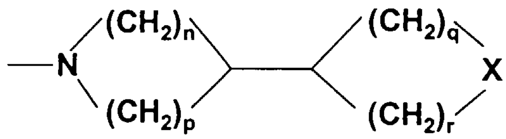

for a compound of the formula (I) is

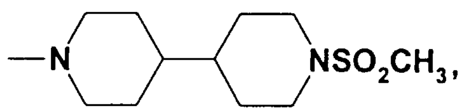

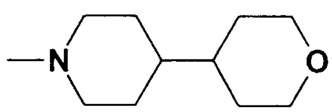

,

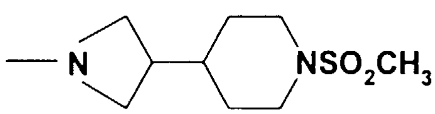

or

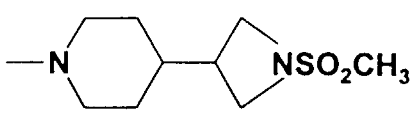

.

**10.** A compound as claimed in any one of the preceding claims of the formula:

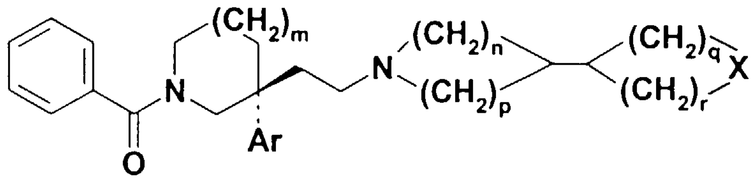

**(IA)**

wherein Ar, X, m, n, p, q and r are as defined in any one of claims 1 to 8.

**11.** A compound as claimed in claim 1 wherein:

Ar is 3,4-dichlorophenyl, X is $NSO_2CH_3$, m is 1 and n, p, q and r are each 2;
Ar is 3,4-dichlorophenyl, X is O, m is 1 and n, p, q and r are each 2;
Ar is 4-chlorophenyl, X is $NSO_2CH_3$, m is 0 and n, p, q and r are each 2;
Ar is 3,4-dichlorophenyl, X is $NSO_2CH_3$, m is 0 and n, p, q and r are each 2;
Ar is 4-chlorophenyl, X is O, m is 0 and n, p, q and r are each 2;
Ar is 3,4-dichlorophenyl, X is $NSO_2CH_3$, m is 1 and n and p are each 1 and q and r are each 2;
Ar is 3,4-difluorophenyl, X is $NSO_2CH_3$, m is 1 and n, p, q and r are each 2;
Ar is 3,4-difluorophenyl, X is O, m is 1 and n, p, q and r are each 2;
Ar is 4-chlorophenyl, X is O, m is 1 and n, p, q and r are each 2;
Ar is 3,4-dichlorophenyl, X is $NSO_2CH_3$, m is 1, n and p are each 2, and q and r are each 1:

or a pharmaceutically acceptable acid addition salt thereof.

**12.** A compound as claimed in claim 11 that has the same stereochemistry as the compound of the formula (IA) defined in claim 10.

**13.** A pharmaceutical composition comprising a compound of the formula (I), or a pharmaceutically acceptable acid addition salt thereof, as claimed in any one of claims 1 to 12, together with a pharmaceutically acceptable diluent or carrier.

**14.** A compound of the formula (I), or a pharmaceutically acceptable acid addition salt or composition thereof, as claimed in any one of claims 1 to 12 and 13, respectively, for use as a medicament.

**15.** The use of a compound of the formula (I), or of a pharmaceutically acceptable acid addition salt or composition thereof, as claimed in any one of claims 1 to 12 and 13, respectively, for the manufacture of a medicament for the treatment of a disease by producing an antagonist effect on a tachykinin acting at the human $NK_1$, $NK_2$ or $NK_3$ receptor, or any combination thereof.

**16.** Use as in claimed in claim 15 where the disease is an inflammatory disease such as arthritis, psoriasis, asthma or inflammatory bowel disease, a central nervous system (CNS) disorder such as anxiety, depression, dementia or psychosis, a gastro-intestinal (GI) disorder such as functional bowel disease, irritable bowel syndrome, gastro-oesophageal reflux, faecal incontinence, colitis or Crohn's disease, an urogenital tract disorder such as incontinence, hyperreflexia or cystitis, a pulmonary disorder such as chronic obstructive airways disease, an allergy such as eczema, contact dermatitis or rhinitis, a hypersensitivity disorder such as to poison ivy, a peripheral neuropathy such as diabetic neuropathy, neuralgia, causalgia, painful neuropathy, a burn, herpetic neuralgia or post-herpetic neuralgia, cough or acute or chronic pain.

**17.** A method of treatment of a human to treat a disease by producing an antagonist effect on a tachykinin acting at the human $NK_1$, $NK_2$ or $NK_3$ receptor, or any combination thereof, which comprises treating said human with an effective amount of a compound of the formula (I) or with a pharmaceutically acceptable acid addition salt or

composition thereof, as claimed in any one of claims 1 to 12 and 13, respectively.

**18.** A method as in claimed in claim 17 where the disease is an inflammatory disease such as arthritis, psoriasis, asthma or inflammatory bowel disease, a central nervous system (CNS) disorder such as anxiety, depression, dementia or psychosis, a gastro-intestinal (GI) disorder such as functional bowel disease, irritable bowel syndrome, gastro-oesophageal reflux, faecal incontinence, colitis or Crohn's disease, an urogenital tract disorder such as incontinence, hyperreflexia or cystitis, a pulmonary disorder such as chronic obstructive airways disease, an allergy such as eczema, contact dermatitis or rhinitis, a hypersensitivity disorder such as to poison ivy, a peripheral neuropathy such as diabetic neuropathy, neuralgia, causalgia, painful neuropathy, a burn, herpetic neuralgia or post-herpetic neuralgia, cough or acute or chronic pain.

**19.** A compound of the formula:

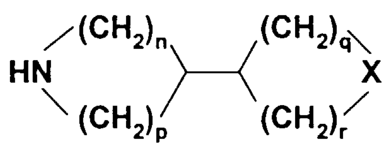

(III)

or an acid addition salt thereof, wherein X, n, p, q and r are as defined in claim 1.

**20.** A compound of the formula:-

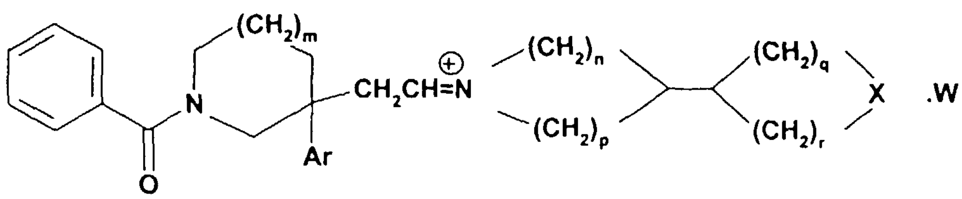

(IV)

where W is an anion derived from an acid, and Ar, X, m, n, p, q, and r are as defined in claim 1.

**21.** A compound of the formula:

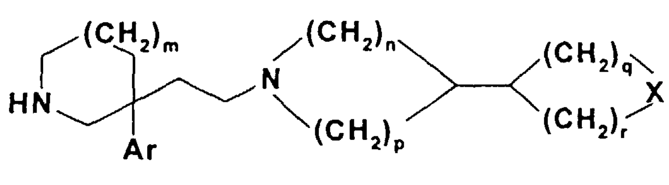

(V)

wherein Ar, X, m, n, p, q, and r are as defined in claim 1.

European Patent Office

# PARTIAL EUROPEAN SEARCH REPORT

which under Rule 45 of the European Patent Convention shall be considered, for the purposes of subsequent proceedings, as the European search report

Application Number EP 99 30 7878

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | WO 97 25322 A (PFIZER) 17 July 1997 (1997-07-17) * page 2, paragraph 1; claims; examples 7,9,10 * --- | 1-21 | C07D211/28 C07D403/14 C07D405/14 A61K31/44 C07D405/06 C07D309/04 |
| Y | EP 0 512 901 A (ELF SANOFI) 11 November 1992 (1992-11-11) * page 3, line 1 - line 24; claims; tables I,,II * --- | 1-21 | |
| A | WO 96 06094 A (MERRELL DOW PHARMACEUTICALS) 29 February 1996 (1996-02-29) * claims; examples * --- | 1-21 | |
| D,A | WO 97 10211 A (SANOFI) 20 March 1997 (1997-03-20) * claims; examples * ----- | 1-21 | |
| | | | TECHNICAL FIELDS SEARCHED (Int.Cl.7) |
| | | | C07D |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claims 17 and 18 are directed to a method of treatment of the human/animal body (Article 52(4) EPC), the search has been carried out and based on the alleged effects of the compound/composition.

| Place of search | Date of completion of the search | Examiner |
|---|---|---|
| MUNICH | 19 January 2000 | Helps, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

## ANNEX TO THE EUROPEAN SEARCH REPORT ON EUROPEAN PATENT APPLICATION NO. EP 99 30 7878

This annex lists the patent family members relating to the patent documents cited in the above-mentioned European search report.
The members are as contained in the European Patent Office EDP file on
The European Patent Office is in no way liable for these particulars which are merely given for the purpose of information.

19-01-2000

| Patent document cited in search report | Publication date | Patent family member(s) | Publication date |
|---|---|---|---|
| WO 9725322 A | 17-07-1997 | AP 709 A | 22-12-1998 |
| | | AU 708282 B | 29-07-1999 |
| | | AU 1195097 A | 01-08-1997 |
| | | BG 102589 A | 30-09-1999 |
| | | BR 9612412 A | 13-07-1999 |
| | | CZ 9802093 A | 14-04-1999 |
| | | EP 0871623 A | 21-10-1998 |
| | | HR 970006 A | 30-06-1998 |
| | | JP 11501667 T | 09-02-1999 |
| | | NO 982651 A | 09-06-1998 |
| | | NZ 324712 A | 28-05-1999 |
| | | PL 327665 A | 21-12-1998 |
| | | ZA 9700047 A | 03-07-1998 |
| EP 512901 A | 11-11-1992 | FR 2676055 A | 06-11-1992 |
| | | AT 181550 T | 15-07-1999 |
| | | AU 652046 B | 11-08-1994 |
| | | AU 1591692 A | 05-11-1992 |
| | | CA 2067877 A | 04-11-1992 |
| | | CS 9201329 A | 18-11-1992 |
| | | DE 69229460 D | 29-07-1999 |
| | | FI 921951 A | 04-11-1992 |
| | | FI 951242 A | 16-03-1995 |
| | | FI 951243 A | 16-03-1995 |
| | | IL 101760 A | 18-02-1997 |
| | | IL 117921 A | 18-02-1997 |
| | | JP 5186425 A | 27-07-1993 |
| | | MX 9202027 A | 01-01-1993 |
| | | NO 178573 B | 15-01-1996 |
| | | NZ 242586 A | 26-10-1995 |
| | | RU 2083574 C | 10-07-1997 |
| | | US 5770735 A | 23-06-1998 |
| | | US 5625060 A | 29-04-1997 |
| | | US 5340822 A | 23-08-1994 |
| WO 9606094 A | 29-02-1996 | AT 177095 T | 15-03-1999 |
| | | AU 693936 B | 09-07-1998 |
| | | AU 3492895 A | 14-03-1996 |
| | | CA 2198084 A | 29-02-1996 |
| | | CN 1158612 A | 03-09-1997 |
| | | DE 69508093 D | 08-04-1999 |
| | | DE 69508093 T | 15-07-1999 |
| | | EP 0777666 A | 11-06-1997 |
| | | ES 2132709 T | 16-08-1999 |
| | | FI 970771 A | 24-02-1997 |
| | | HU 76644 A | 28-10-1997 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 99 30 7878

This annex lists the patent family members relating to the patent documents cited in the above-mentioned European search report.
The members are as contained in the European Patent Office EDP file on
The European Patent Office is in no way liable for these particulars which are merely given for the purpose of information.

19-01-2000

| Patent document cited in search report | Publication date | Patent family member(s) | Publication date |
|---|---|---|---|
| WO 9606094 A | | JP 10504580 T | 06-05-1998 |
| | | NO 970831 A | 18-04-1997 |
| | | NZ 292683 A | 28-07-1998 |
| | | ZA 9507033 A | 16-04-1996 |
| WO 9710211 A | 20-03-1997 | FR 2738819 A | 21-03-1997 |
| | | AU 6992596 A | 01-04-1997 |
| | | BR 9610081 A | 05-01-1999 |
| | | CA 2232007 A | 20-03-1997 |

EPO FORM P0459

For more details about this annex : see Official Journal of the European Patent Office, No. 12/82